(19) Europäisches Patentamt
European Patent Office
Office européen des brevets

(11) EP 3 009 419 A1

(12) # EUROPEAN PATENT APPLICATION
published in accordance with Art. 153(4) EPC

(43) Date of publication:
**20.04.2016 Bulletin 2016/16**

(21) Application number: **14810866.5**

(22) Date of filing: **13.06.2014**

(51) Int Cl.:
***C07C 213/00*** (2006.01) ***B01J 31/02*** (2006.01)
***B01J 31/22*** (2006.01) ***C07B 53/00*** (2006.01)
***C07C 217/64*** (2006.01) ***C07C 231/18*** (2006.01)
***C07C 233/18*** (2006.01) ***C07C 253/30*** (2006.01)
***C07C 255/37*** (2006.01) ***C07C 269/06*** (2006.01)
***C07C 271/16*** (2006.01) ***C07D 209/48*** (2006.01)
***C07F 7/18*** (2006.01) ***C07B 61/00*** (2006.01)

(86) International application number:
**PCT/JP2014/065771**

(87) International publication number:
**WO 2014/200094 (18.12.2014 Gazette 2014/51)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.06.2013 JP 2013125119**

(71) Applicant: **Nissan Chemical Industries, Ltd.**
**Chiyoda-ku**
**Tokyo 101-0054 (JP)**

(72) Inventors:
- **KAWANAMI, Hirotaka**
**Sanyo-Onoda-shi**
**Yamaguchi 756-0093 (JP)**
- **NISHINO, Yukihiro**
**Sanyo-Onoda-shi**
**Yamaguchi 756-0093 (JP)**
- **MURASE, Shota**
**Sanyo-Onoda-shi**
**Yamaguchi 756-0093 (JP)**

(74) Representative: **Hoffmann Eitle**
**Patent- und Rechtsanwälte PartmbB**
**Arabellastraße 30**
**81925 München (DE)**

(54) ## PRODUCTION METHOD FOR OPTICALLY ACTIVE ALCOHOL COMPOUND

(57) There is provided a method for stereoselectively producing an optically active alcohol compound. The optically active alcohol compound of Formula (8) can be produced in high yield and high selectivity from the compound of Formula (3), and the production method that is useful industrially and the intermediates therefor can be provided.

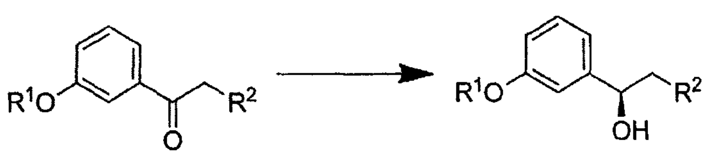

(3) (8)

In formulae, $R^1$ is a hydrogen atom, $C_{1-6}$ alkyl, $C_{1-6}$ alkyl optionally substituted with $R^3$, or the like, $R^2$ is cyano or $-CH_2N(R^5)R^4$, and $R^3$ is $C_{3-8}$ cycloalkyl.

EP 3 009 419 A1

**Description**

TECHNICAL FIELD

The present invention relates to a method for producing an optically active alcohol compound.

BACKGROUND ART

It is conventionally known that optically active alcohol compounds have been used as production intermediates for pharmaceuticals, agricultural chemicals, and electronic materials. In particular, 3-amino-1-(3-alkoxy)phenylpropan-1-ol derivatives of Formula (1) have been known as important compounds for medical products for eye disease (for example, see Patent Document 1).

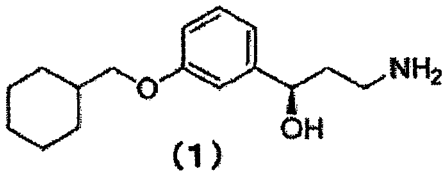

(1)

As the method for producing the compound (1), a method for producing 3-amino-1-(3-alkoxy) phenylpropan-1-ol derivatives by carrying out a reduction reaction of 3-amino-1-(3-alkoxy)phenylpropan-1-one of Formula (2) in the presence of an asymmetric reduction catalyst such as (-)-B-chlorodiisopinocampheylborane[(-)-DIPCl] has been known (for example, refer to Patent Document 1).

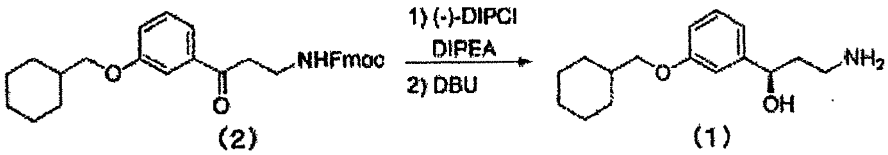


(2) (1)

(in the above formula, Fmoc is 9-fluorenylmethyloxycarbonyl, DIPEA is N,N-diisopropylethylamine, and DBU is diazabicycloundecene.)

Prior Art Documents

Patent Documents

Patent Document 1: International Publicaiton WO 2009/045479 Pamphlet

SUMMARY OF THE INVENTION

Problem to be Solved by the Invention

In the method described in Patent Document 1, the stereoselectivity of the compound of Formula (1) obtained from the reduction reaction of the compound of Formula (2) is 96.1:3.9. This selectivity is insufficient for using the compound of Formula (1) as a medical product. An additional operation for improving the optical purity is required and thus this method is hardly an industrially suitable production method.

Means for Solving the Problem

In view of the above circumstances, the inventors of the present invention have extensively studied for a purpose of providing industrially useful production method of the optically active alcohol compound in higher yield and higher selectivity. As a result, the inventors of the present invention have found a production method in which the optically active alcohol compound can be obtained in extremely high stereoselectivity and high yield and have accomplished the present invention.

Namely, the present invention relates to the following aspects [1] to [21].

[1] A method for producing an optically active alcohol compound of Formula (8):

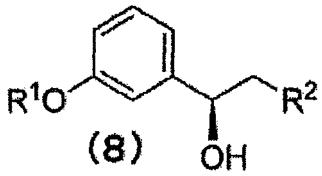

(8)

[in the formula, $R^1$ is a hydrogen atom, $C_{1-6}$ alkyl, $(C_{1-6})$ alkyl optionally substituted with $R^3$, $-C(O)R^8$, or $-Si(R^{12a})(R^{12b})R^{12}$; and
$R^2$ is cyano or $-CH_2N(R^5)R^4$;
$R^3$ is $C_{1-6}$ alkoxy, phenyl, or $C_{3-8}$ cycloalkyl;
$R^4$ is a hydrogen atom, $-C(O)R^6$, or $-C(O)OR^7$;
$R^5$ is a hydrogen atom or $C_{1-6}$ alkyl, or $R^5$ optionally forms a 5- to 7-membered ring together with a nitrogen atom to which $R^4$ and $R^5$ are bonded by forming a $C_{4-6}$ alkylene chain together with $R^4$, and in this case the alkylene chain is optionally substituted with one or more selected from the group consisting of $C_{1-6}$ alkyl, $(C_{1-6})$ alkyl optionally substituted with Y, phenyl, and an oxo group, or the alkylene chain optionally forms phenyl together with carbon atoms to which two substituents each bond when the two substituents exist at adjacent positions on the alkylene chain;
$R^6$ is $C_{1-6}$ alkyl, $(C_{1-6})$ alkyl optionally substituted with a halogen atom, or phenyl; $R^7$ is $C_{1-6}$ alkyl or $-CH_2R^{13}$;
$R^8$ is a hydrogen atom, $C_{1-6}$ alkyl, $(C_{1-6})$ alkyl optionally substituted with a halogen atom, phenyl, or phenyl substituted with (Z)p;
$R^{12}$, $R^{12a}$, and $R^{12b}$ each are independently $C_{1-6}$ alkyl or phenyl;
$R^{13}$ is phenyl or 9-fluorenyl; Y is a halogen atom;
Z is a halogen atom or $C_{1-6}$ alkoxy; and
p is an integer of 1, 2, 3, 4, or 5];
the method characterized by comprising the step of:

reacting a compound of Formula (3):

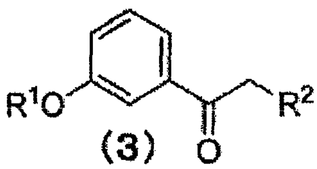

(3)

(in the formula, $R^1$ and $R^2$ are the same meaning as Formula (8));

with a reducing agent in the presence of an optically active ruthenium catalyst selected from the group consisting of a compound of Formula (4):

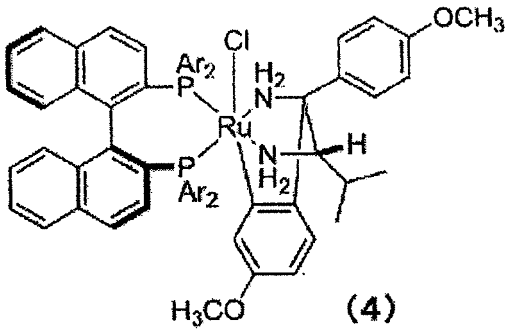

(4)

(in the formula, Ar is 3,5-dimethylphenyl), a compound of Formula (5):

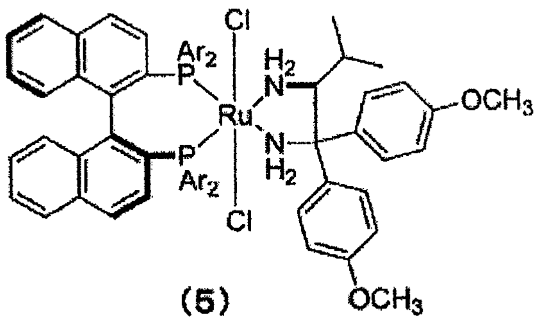

(5)

(in the formula, Ar is 3,5-dimethylphenyl), and a compound of Formula (6):

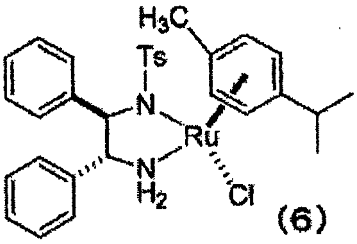

(6)

(in the formula, Ts is paratoluenesulfonyl) or an optically active oxazaborolidine compound of Formula (7),

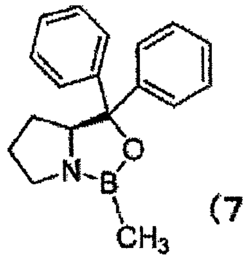

(7)

[2] The method for producing the optically active alcohol compound described in the aspect [1], in which
$R^1$ is a hydrogen atom, ($C_{1-6}$) alkyl optionally substituted with $R^3$, -C(O)$R^8$-, or
-Si($R^{12a}$)($R^{12b}$)$R^{12}$.
[3] The method for producing the optically active alcohol compound described in the aspect [2], in which the reaction is carried out in the presence of the optically active ruthenium catalyst of Formula (4).
[4] The method for producing the optically active alcohol compound described in the aspect [3], in which
$R^2$ is -$CH_2N(R^5)R^4$;
$R^4$ is -C(O)$R^6$ or -C(O)O$R^7$;
$R^6$ is $C_{1-6}$ alkyl or ($C_{1-6}$) alkyl optionally substituted with a halogen atom; and
$R^8$ is a hydrogen atom, $C_{1-6}$ alkyl, or ($C_{1-6}$) alkyl optionally substituted with a halogen atom.
[5] The method for producing the optically active alcohol compound described in the aspect [4], in which
$R^1$ is ($C_{1-6}$) alkyl optionally substituted with $R^3$, or -Si($R^{12a}$)($R^{12a}$)$R^{12}$;
$R^2$ is -$CH_2N(R^5)R^4$;

$R^3$ is phenyl, or $C_{3-8}$ cycloalkyl;
$R^4$ is -C(O)$R^6$ or -C(O)O$R^7$;
$R^5$ is a hydrogen atom, or $R^5$ optionally forms a 5-membered ring together with a nitrogen atom to which $R^4$ and $R^5$ are bonded by forming a $C_4$ alkylene chain together with $R^4$, and in this case the alkylene chain is optionally substituted with an oxo group, or the alkylene chain optionally forms phenyl together with the carbon atoms to which two substituents each bond when the two substituents exist at adjacent positions on the alkylene chain;
$R^6$ is ($C_{1-6}$) alkyl optionally substituted with a halogen atom; and
$R^{13}$ is 9-fluorenyl.

[6] The method for producing the optically active alcohol compound described in the aspect [5], in which
$R^1$ is ($C_{1-6}$) alkyl optionally substituted with $R^3$;
$R^3$ is $C_{3-8}$ cycloalkyl;
$R^4$ is $-C(O)OR^7$;
$R^5$ is a hydrogen atom; and
$R^7$ is $C_{1-6}$ alkyl.

[7] The method for producing the optically active alcohol compound described in any one of the aspects [3] to [6], in which the reducing agent is hydrogen gas.

[8] The method for producing the optically active alcohol compound described in the aspect [2], in which the reaction is carried out in the presence of the optically active ruthenium catalyst of Formula (6).

[9] The method for producing the optically active alcohol compound described in the aspect [8], in which
$R^6$ is $C_{1-6}$ alkyl or ($C_{1-6}$) alkyl optionally substituted with a halogen atom.

[10] The method for producing the optically active alcohol compound described in the aspect [9], in which
$R^1$ is a hydrogen atom, ($C_{1-6}$)alkyl optionally substituted with $R^3$, $-C(O)R^8$, or $-Si(R^{12a})(R^{12b})R^{12}$;
$R^5$ is a hydrogen atom, or $R^5$ optionally forms a 5-membered ring together with a nitrogen atom to which $R^4$ and $R^5$ are bonded by forming a $C_4$ alkylene chain together with $R^4$, and in this case the alkylene chain is optionally substituted with an oxo group, or the alkylene chain optionally forms phenyl together with the carbon atoms to which two substituents each bond when the two substituents exist at adjacent positions on the alkylene chain;
$R^6$ is ($C_{1-6}$) alkyl optionally substituted with a halogen atom;
$R^7$ is $C_{1-6}$ alkyl;
$R^8$ is phenyl; and
$R^{13}$ is 9-fluorenyl.

[11] The method for producing the optically active alcohol compound described in the aspect [10], in which
$R^1$ is $C_{1-6}$ alkyl optionally substituted with $R^3$;
$R^2$ is cyano; and
$R^3$ is $C_{3-8}$ cycloalkyl.

[12] The method for producing the optically active alcohol compound described in any one of the aspects [8] to [11], in which the reducing agent is formic acid.

[13] A compound of Formula (3'):

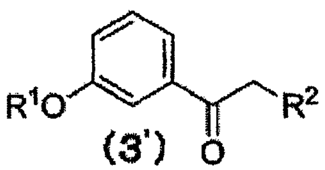

(in the formula, $R^1$ is $C_1$ alkyl substituted with $R^3$; $R^2$ is cyano; and
$R^3$ is cyclohexyl).

[14] A method for producing a compound of Formula (3"):

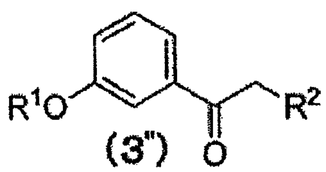

(in the formula, $R^1$ is a cyclohexylmethyl group; and
$R^2$ is $-CH_2NHC(O)O(C_4H_9\text{-}t)$ or $-CH_2NHCOCF_3$);
the method characterized by comprising the step of:

reacting an alcohol compound of Formula (44):

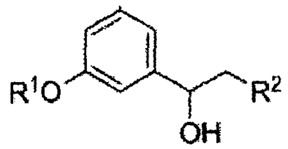

(44)

(in the formula, $R^1$ and $R^2$ are the same meaning as Formula (3")) with an oxidizing agent in the presence of N-hydroxy-2-azaadamantane of Formula (43):

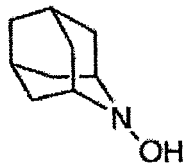

(43)

[15] The method for producing the optically active alcohol compound described in any one of the aspects [1] to [12], in which
$R^1$ is methyl substituted with one $R^3$; and
$R^3$ is cyclohexyl.
[16] The method for producing the optically active alcohol compound described in any one of the aspects [1] to [12], in which
$R^1$ is methyl substituted with one $R^3$; and
$R^3$ is phenyl.
[17] The method for producing the optically active alcohol compound described in any one of the aspects [1] to [12], in which
$R^1$ is $-Si(R^{12a})(R^{12b})R^{12}$.
[18] The method for producing the optically active alcohol compound described in any one of the aspects [1] to [12], in which
$R^1$ is methyl substituted with one $R^3$; and $R^3$ is $C_{1-6}$ alkoxy.
[19] The method for producing the optically active alcohol compound described in any one of the aspects [1] to [12], in which
$R^2$ is $-CH_2N(R^5)R^4$;
$R^4$ is $-C(O)R^6$ or $-C(O)OR^7$;
$R^5$ is a hydrogen atom;
$R^6$ is $(C_{1-6})$ alkyl optionally substituted with a halogen atom; and
$R^7$ is $C_{1-6}$ alkyl.
[20] The method for producing the optically active alcohol compound described in any one of the aspects [1] to [12], in which
$R^2$ is cyano.
[21] The method for producing the optically active alcohol compound described in any one of the aspects [1] to [12], in which
$R^2$ is cyano or $-CH_2N(R^5)R^4$.

Effects of the Invention

According to the present invention, the optically active alcohol compound of Formula (8) that is useful as medical products can be produced in high selectivity and in large amounts and thus the present invention has a high utility value as an industrial production method.

MODES FOR CARRYING OUT THE INVENTION

Hereinafter, the present invention will be described in further detail.
In this specification, "n-" means normal; "i-" means iso; "s-" means secondary; "t-" or "-t" means tertiary; "c-"

means cyclo, "o-" means ortho; "m-" means meta; "p-" means para; "Bu" means butyl; "Bz" means benzoyl; "Bn" means benzyl; "Fmoc" means 9-fluorenylmethyloxycarbonyl; "Boc" means tert-butoxycarbonyl; "TBS" means a tert-butyldimethylsilyl group; "DBU" means diazabicycloundecene; and "MOM" means methoxymethyl.

In this specification, the compound of Formula (X) is described in an abbreviated manner as the "compound (X)".

Specific examples of each substituent described in this specification are described below.

In this specification, $C_{a-b}$ alkyl is a linear or branched hydrocarbon group in which the number of carbon atoms is a to b. Specific examples of the $C_{a-b}$ alkyl may include methyl group, ethyl group, n-propyl group, i-propyl group, n-butyl group, i-butyl group, t-butyl group, s-butyl group, n-pentyl group, 1-methylbutyl group, 2-methylbutyl group, 3-methylbutyl group, 1-ethylpropyl group, 1,1-dimethylpropyl group, 1,2-dimethylpropyl group, neopentyl group, n-hexyl group, 1-methylpentyl group, 2-methylpentyl group, 3-methylpentyl group, 4-methylpentyl group, 1-ethylbutyl group, 2-ethylbutyl group, 1,1-dimethylbutyl group, 1,2-dimethylbutyl group, 1,3-dimethylbutyl group, 2,2-dimethylbutyl group, 2,3-dimethylbutyl group, 3,3-dimethylbutyl group, 1,1,2-trimethylpropyl group, 1-ethyl-1-methylpropyl group, 1-ethyl-2-methylpropyl group, 1-heptyl group, 1-octyl group, 1-nonyl group, 1-decanyl group, 1-undecanyl group, and 1-dodecanyl group. The group is selected in a range of the specified number of carbon atoms.

In this specification, $C_{a-b}$ cycloalkyl is a cyclic hydrocarbon group in which the number of carbon atoms is a to b, and a single ring structure or a multi-ring structure can be formed. Each ring may be optionally substituted with an alkyl group in a range of the specified carbon number. Specific examples of the $C_{a-b}$ cycloalkyl may include cyclopropyl group, 1-methylcyclopropyl group, 2-methylcyclopropyl group, 2,2-dimethyl-cyclopropyl group, cyclobutyl group, cyclopentyl group, and cyclohexyl group. The group is selected in a range of the specified number of carbon atoms.

In this specification, $C_{a-b}$ alkoxy is an alkyl-O- group, in which the alkyl is the same meaning as described above and the number of carbon atoms is a to b. Specific examples of the $C_{a-b}$ alkoxy may include methoxy group, ethoxy group, n-propyloxy group, i-propyloxy group, n-butyloxy group, s-butyloxy group, i-butyloxy group, t-butyloxy group, n-pentyloxy group, and n-hexyloxy group. The group is selected in a range of the specified number of carbon atoms.

In this specification, $(C_{a-b})$ alkyl optionally substituted with $R^3$ is an alkyl group having the number of carbon atoms of a to b, having the same meaning as described above, and formed by substituting the hydrogen atom bonded to the carbon atom with an optional substituent $R^3$ at any position and any number. The group is selected in a range of the specified number of carbon atoms. In this case, when two or more of the substituents $R^3$ exist on each $(C_{a-b})$ alkyl group, each $R^3$ may be the same as or different from each other.

In this specification, $-N(R^5)R^4$ described by $R^5$ optionally forms a 5- to 7-membered ring together with a nitrogen atom to which $R^4$ and $R^5$ are bonded by forming a $C_{4-6}$ alkylene chain together with $R^4$, and in this case, the alkylene chain is optionally substituted with one or more selected from the group consisting of $C_{1-6}$ alkyl, $(C_{1-6})$ alkyl optionally substituted with Y, phenyl, and an oxo group, or the alkylene chain optionally forms phenyl together with the carbon atoms to which two substituents each bond when the two substituents exist at adjacent positions on the alkylene chain" may include the following groups.

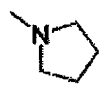
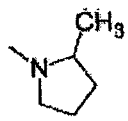
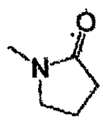
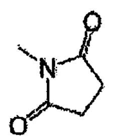
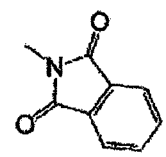

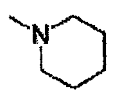
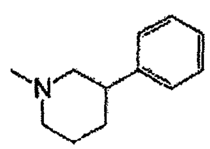
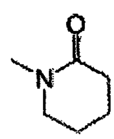
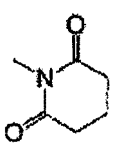
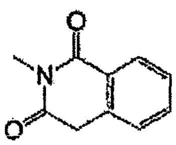

The method for producing the optically active alcohol compound (8) made from the compound (3) as the starting material in the present invention will be described in detail.

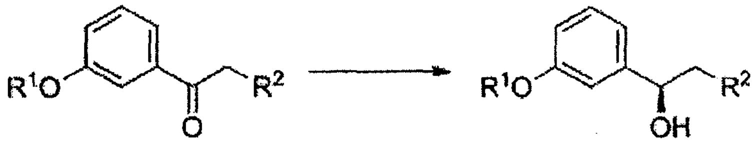

(3) (8)

(i) The optically active alcohol compound (8) (in the formula, $R^1$ and $R^2$ mean the same as the formula described above) can be synthesized by reacting the compound (3) (in the formula, $R^1$ and $R^2$ are the same meaning as the formula described above) with the reducing agent in the presence of the asymmetric reduction catalyst.

As the usable asymmetric reduction catalyst, for example, an optically active ruthenium catalyst can be used. Preferable examples of the optically active ruthenium may include commercially available RUCY (registered trademark)-XylBINAP (sold by TAKASAGO INTERNATIONAL CORPORATION) of Formula (4):

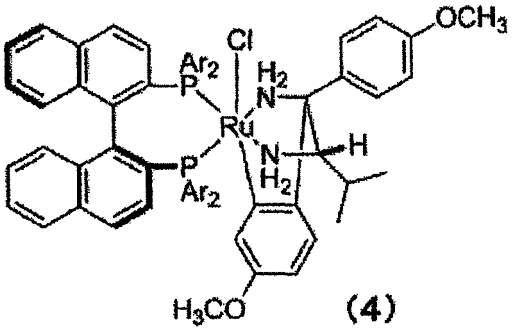

(4)

(in the formula, Ar is 3,5-dimethylphenyl) or $RuCl_2$[(S)-xylbinap][(S)-daipen] (sold by TAKASAGO INTERNATIONAL CORPORATION) of Formula (5):

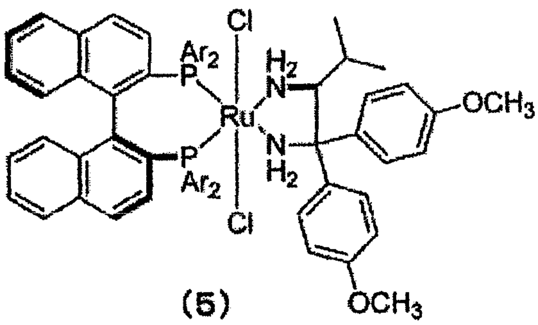

(5)

(in the formula, Ar is 3,5-dimethylphenyl).

The amount of the asymmetric reduction catalyst to be used is 1/100,000 mol% to 100 mol% and preferably 0.01 mol% to 5 mol% relative to the compound (3).

A base can be added for the reaction. Examples of the base to be used may include alkali metal salts of alcohols. Specific Examples of the alkali metal salts of alcohols may include $CH_3ONa$, $C_2H_5ONa$, and t-BuOK and t-BuOK is the most preferable.

The amount of the base to be added may be the same amount or more relative to the asymmetric reduction catalyst to be used and is preferably 10 to 20 equivalents relative to the catalyst.

The solvent used for the reaction is not limited as long as the solvent does not inhibit the reaction progress. Examples of the solvent may include water, aprotic polar organic solvents (such as N,N-dimethylformamide, dimethylsulfoxide, N,N-dimethylacetamide, tetramethylurea, sulfolane, N-methyl-2-pyrrolidone, and N,N-dimethylimidazolidinone), ether solvents (such as diethyl ether, diisopropyl ether, t-butyl methyl ether, tetrahydrofuran, and dioxane), aliphatic hydrocarbon solvents (such as pentane, n-hexane, c-hexane, octane, decane, decalin, and petroleum ether), aromatic hydrocarbon solvents (such as benzene, chlorobenzene, o-dichlorobenzene, nitrobenzene, toluene, xylene, mesitylene, and tetralin), halogenated hydrocarbon solvents (such as chloroform, dichloromethane, dichloroethane, and carbon tetrachloride), lower aliphatic acid ester solvents (such as methyl acetate, ethyl acetate, butyl acetate,

and methyl propionate), alkoxy alkane solvents (such as dimethoxyethane and diethoxyethane), alcohol solvents (such as methanol, ethanol, 1-propanol, and 2-propanol), and carboxylic acid solvents (such as acetic acid). Among them, the alcohol solvents are preferable. The alcohol solvents to be used may be used singly or in combination of two or more of them. The mixture of ethanol and 2-propanol is preferably used. The mixture can be used in any mixing ratio and the most preferable mixing ratio is ethanol:2-porpanol = 1:1 in a mass ratio.

The reaction temperature is preferably about 10°C to about 40°C and more preferably about 20°C to about 30°C.

The reducing agent that can be used in this reaction is not particularly limited as long as the reducing agent is used as a reagent. Examples of the reducing agent may include hydrogen gas.

The reaction can be carried out at any hydrogen pressure. The preferable hydrogen pressure is in a range of 0.1 MPa to 1.0 MPa and more preferably 0.5 MPa.

(ii) The optically active alcohol compound (8) can be synthesized by reacting the compound (3) with the reducing agent in the presence of a hydrogen transfer type asymmetric reduction catalyst.

Examples of the hydrogen transfer type asymmetric reduction catalyst that can be used in the reaction may include commercially available RuCl[(R,R)-Tsdpen](p-cymene) (sold by KANTO CHEMICAL CO., INC.) of Formula (6).

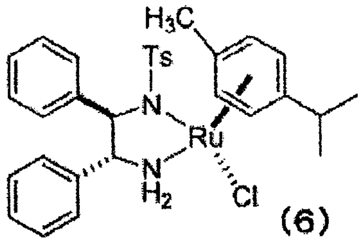

(6)

The amount of the hydrogen transfer type asymmetric reduction catalyst to be used is 1/100,000 mol% to 100 mol% and preferably 0.01 mol% to 20 mol% relative to the compound (3).

In this reaction, hydrogen gas is not required to be fed as the reducing agent and a reaction reagent can be used as a hydrogen source. Examples of the reaction reagent to be the hydrogen source may include a mixed solution of formic acid and triethylamine, and 2-propanol. In this reaction, the mixed solution of formic acid and triethylamine is preferable. The amount of formic acid and triethylamine to be used may be any amount relative to the compound (3). Formic acid may be used in an amount of 3 equivalents to 9 equivalents, and triethylamine may be used in an amount of 2 equivalents to 8 equivalents. The most desirable amounts are 3.1 equivalents of formic acid and 5.2 equivalents of triethylamine.

The mixed solution of formic acid and triethylamine described above can be also used as a reaction solvent. Another solvent, however, can be added and used as long as this solvent does not inhibit the reaction progress. This solvent is not particularly limited and examples of this solvent may include water, aprotic polar organic solvents (such as N,N-dimethylformamide, dimethylsulfoxide, N,N-dimethylacetamide, tetramethylurea, sulfolane, N-methyl-2-pyrrolidone, and N,N-dimethylimidazolidinone), ether solvents (such as diethyl ether, diisopropyl ether, t-butyl methyl ether, tetrahydrofuran, and dioxane), aliphatic hydrocarbon solvents (such as pentane, n-hexane, c-hexane, octane, decane, decalin, and petroleum ether), aromatic hydrocarbon solvents (such as benzene, chlorobenzene, o-dichlorobenzene, nitrobenzene, toluene, xylene, mesitylene, and tetralin), halogenated hydrocarbon solvents (such as chloroform, dichloromethane, dichloroethane, and carbon tetrachloride), lower aliphatic acid ester solvents (such as methyl acetate, ethyl acetate, butyl acetate, and methyl propionate), and alkoxy alkane solvents (such as dimethoxyethane and diethoxyethane). In this reaction, the solvent to be used is preferably the aprotic polar organic solvents. Among them, dimethylformamide is the most preferable. The amount of the solvent to be used can be any amount. The most appropriate amount is an amount two times larger than the amount of the compound (3) in mass.

The reaction temperature is preferably 20°C to 30°C.

(iii) The optically active alcohol compound (8) can be synthesized by reacting the compound (3) with the reducing agent in the presence of an optically active oxazaborolidine compound.

Examples of the usable optically active oxazaborolidine compound may include a commercially available optically active oxazaborolidine compound of Formula (7).

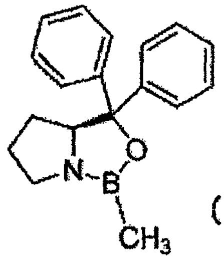

(7)

The amount of the optically active oxazaborolidine compound to be used 1/100,000 mol% to 100 mol% and preferably 1 mol% to 30 mol% relative to the compound (3).

The reducing agent that can be used in this reaction is not particularly limited as long as the reducing agent is used as a reagent. Examples of the reducing agent may include a borane-dimethylsulfide complex and a borane-tetrahydrofuran complex.

The amount of the reducing agent to be used may be any amount of 0.6 equivalent to 2 equivalents relative to the compound (3).

The solvent used for the reaction is not limited as long as the solvent does not inhibit the reaction. Examples of the solvent may include ether solvents (such as diethyl ether, diisopropyl ether, t-butyl methyl ether, tetrahydrofuran, and dioxane), aliphatic hydrocarbon solvents (such as pentane, n-hexane, c-hexane, octane, decane, decalin, and petroleum ether), aromatic hydrocarbon solvents (such as benzene, chlorobenzene, o-dichlorobenzene, nitrobenzene, toluene, xylene, mesitylene, and tetralin), and halogenated hydrocarbon solvents (such as chloroform, dichloromethane, dichloroethane, and carbon tetrachloride). The aromatic hydrocarbon solvents are preferable and, among them, toluene is the most preferable.

With respect to the reaction temperature, the reaction can be carried out at any temperature of -50°C to 40°C. In the present invention, however, the reaction temperature is preferably about 20°C.

With respect to the production method of the invention in the present specification, the compound (3), which is a ketone derivative used for the reduction reaction, includes known compounds. Although some of the ketone derivatives can be obtained as reagents, the method for producing the compound (3) will be described below.

Among the compounds (3), a method for synthesizing the following specific compound (14) will be described below.

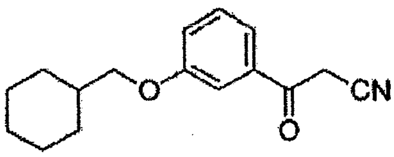

(14)

The compound (14) can be synthesized by oxidizing the compound (13) being the known compound.

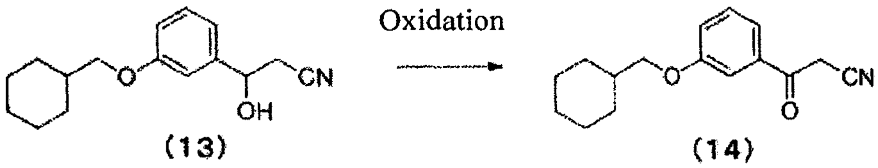


(13) (14)

As described below, for example, a method of using an oxidizing agent in the presence of potassium 2-iodobenzenesulfonate (9) as an oxidation catalyst can be selected as the method.

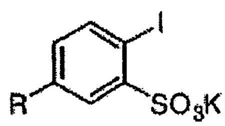

(9)

(In the formula, R is a hydrogen atom or $C_{1-10}$ alkyl)

The substituent R in potassium 2-iodo-benzenesulfonate (9) is desirably a hydrogen atom or a methyl group. Potassium 2-iodo-benzenesulfonate (9) is a known compound and some of the compounds can be obtained as a reagent from JUNSEI CHEMICAL CO., LTD. or other suppliers.

Potassium 2-iodo-benzenesulfonate (9) is preferably used in a range of an amount of 0.01 mol% to 20 mol% relative to the compound (13).

The oxidizing agent is not particularly limited as long as the oxidizing agent can oxidize the iodine group of the compound (9) to pentavalent iodine. The oxidizing agent may be either an inorganic oxidizing agent or an organic oxidizing agent. In the present invention, among them, potassium monopersulfate compound salt [OXONE (registered trademark)] is preferable. The amount of OXONE (registered trademark) to be used is not particularly limited. In the present invention, the amount is preferably 1.5 to 2.0 times larger in mole than the amount of the compound (13).

With respect to the reaction temperature of the oxidation reaction, the reaction can be carried out at 20°C to 100°C as needed. In the present invention, the reaction temperature is more preferably 70°C.

The solvent used for the oxidation reaction is not limited as long as the solvent does not inhibit the reaction progress. Examples of the solvent may include ether solvents (such as diethyl ether, diisopropyl ether, t-butyl methyl ether, tetrahydrofuran, and dioxane), aliphatic hydrocarbon solvents (such as pentane, n-hexane, c-hexane, octane, decane, decalin, and petroleum ether), aromatic hydrocarbon solvents (such as benzene, chlorobenzene, o-dichlorobenzene, nitrobenzene, toluene, xylene, mesitylene, and tetralin), halogenated hydrocarbon solvents (such as chloroform, dichloromethane, dichloroethane, and carbon tetrachloride), ketone solvents (such as acetone, methyl ethyl ketone, methyl butyl ketone, and methyl isobutyl ketone), lower aliphatic acid ester solvents (such as methyl acetate, ethyl acetate, butyl acetate, and methyl propionate), alkoxy alkane solvents (such as dimethoxyethane and diethoxyethane), and nitrile solvents (such as acetonitrile, propionitrile and butyronitrile). Among them, for example, ethyl acetate and acetonitrile are preferable.

Some of the compounds (3) except the compound (14) are known compounds that are available as reagents. The other compounds of the compound (3) can also be synthesized in accordance with the method described above.

Among the compounds (3), for example, a method for producing the compound (2), in which $R^1$ is a cyclohexylmethyl group and $R^2$ is a $CH_2NHFmoc$ group, is described in Patent Document 1. More specifically, the compound (2) can be produced by the reaction using a manganese compound as an oxidizing agent to the compound (42). The compound (2) can also be produced from the compound (42) by the reaction using oxidizing agent in the presence of the N-hydroxyl compound of Formula (43).

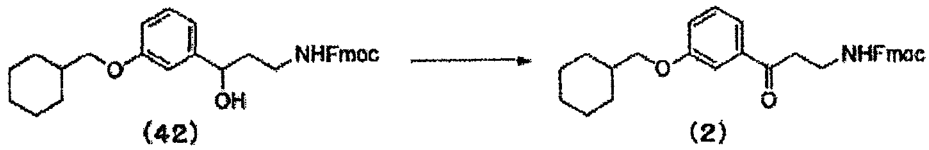

(42) (2)

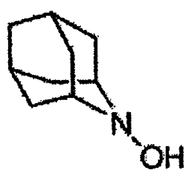

(43)

The N-hydroxyl compound (43) can be obtained as AZADOL [(registered trademark), sold by Wako Pure Chemical Industries, Ltd.] being the commercial product.

The amount of the N-hydroxyl compound (43) to be used is preferably 0.1 mol% to 50 mol% and more preferably 1 mol% to 10 mol% relative to the alcohol compound (compound (42)) being the substrate.

Examples of the oxidizing agent in the above reaction may include organic or inorganic compounds containing an oxygen atom. Typical examples may include peroxygen acids such as peracetic acid, hydrogen peroxide ($H_2O_2$), hypohalites, halites, halides, diacetoxyiodoallenes, oxygen, or a combination thereof. Alkali metal hypohalites and alkaline earth metal hypohalites are preferable as the hypohalites and examples of the hypohalites may include LiOCl, NaOCl, KOCl, LiOBr, NaOBr, and KOBr. Specifically, the oxidizing agent is preferably the alkali metal hypohalite. Among them, sodium hypochlorite (NaOCl) is preferable in the present invention.

With respect to the reaction temperature, the reaction can be carried out at room temperature. The reaction, however, can be carried out at a temperature in a range of 10°C to 40°C, in a range of 0°C to 100°C, and at -10°C to 200°C as needed. With respect to the reaction pressure, normal pressure (atmospheric pressure) can be sufficient for the reaction. The reaction, however, can be carried out in a reduced pressure state and a pressurized state in a range of 0.01 MPa to 10 MPa.

The reaction time is 1 minute to 100 hours and preferably 5 minutes to 24 hours.

The solvent used for this oxidation reaction is not limited as long as the solvent does not inhibit the reaction progress. Examples of the solvent may include water, aprotic polar organic solvents (such as dimethylformamide, dimethylsulfoxide, dimethylacetamide, tetramethylurea, sulfolane, N-methyl-2-pyrrolidone, and N,N-dimethyl imidazolidinone), ether solvents (such as diethyl ether, diisopropyl ether, t-butyl methyl ether, tetrahydrofuran, and dioxane), aliphatic hydrocarbon solvents (such as pentane, hexane, c-hexane, octane, decane, decalin, and petroleum ether), aromatic hydrocarbon solvents (such as benzene, chlorobenzene, o-dichlorobenzene, nitrobenzene, toluene, xylene, mesitylene, and tetralin), halogenated hydrocarbon solvents (such as chloroform, dichloromethane, dichloroethane, and carbon tetrachloride), ketone solvents (such as acetone, methyl ethyl ketone, methyl butyl ketone, and methyl isobutyl ketone), lower aliphatic acid ester solvents (such as methyl acetate, ethyl acetate, butyl acetate, and methyl propionate), alkoxy alkane solvents (such as dimethoxyethane and diethoxyethane), nitrile solvents (such as acetonitrile, propionitrile and butyronitrile), and carboxylic acid solvents (such as acetic acid). Among them, for example, toluene is preferable.

To the reaction liquid mixture, a buffering agent such as an inorganic salt or an organic salt can be added. Examples of the buffering agent to be used may include carbonates of alkali metals or alkaline earth metals, hydrogen carbonates of alkali metals or alkaline earth metals, hydroxides of alkali metals or alkaline earth metals, and phosphates of alkali metals or alkaline earth metals. The buffering agent is desirably the hydrogen carbonates of alkali metals or alkaline earth metals.

When toluene is used as the reaction solvent, an aqueous solution of sodium hypochlorite being an oxidizing agent can be used and oxidation can be carried out in a two-phase reaction system.

Examples

Hereinafter, the present invention will be more specifically described with reference to Examples and Comparative Examples. The present invention, however, is not limited to the following Examples. The following devices and conditions were used for measurements using nuclear magnetic resonance spectrum ($^1$H-NMR), liquid chromatography (LC), and liquid chromatography-mass measurement spectrometry (LC-MS).

"OXONE" used in Examples is a "potassium monopersulfate compound salt" and registered trademark.

In the description of Examples, for example, a "solution of 10 g of a compound (X) in 100 g of an organic solvent" means the solution in which 10 g of the compound (X) is dissolved or dispersed in 100 g of the organic solvent.

[1] $^1$H-NMR Model: AVANCE III 500 (manufactured by BRUKER Corporation, 500 MHz) Measurement solvent: $CDCl_3$

[2] LC (liquid chromatography)

(1) LC conditions example 1: Method name LC-1 (used for measurement of conversion of reaction and quantitative analysis)

LC: Shimadzu 20A (manufactured by Shimadzu Corporation)
Column: YMC-Pack Pro C18 RS
250 × 4.6 mm, I. D. 5 μm
Oven Temperature: 30°C
Eluent: $CH_3CN$, $H_2O$
$CH_3CN$ = 10% (0 min.) → 100% (15 min.) → 100% (25 min.) → 10% (25.01 min.) → 10% (30 min.)
Time program in the parentheses is a total time from the start of analysis.
Flow rate: 1.2 mL/min.
Detector: UV 210 nm

(2) LC conditions example 2: Method name LC-2 (used for analysis of optical purity)

LC: Agilent 1200 (manufactured by Agilent Technologies Co., Ltd.)
Column: CHIRALPAK IA
250 × 4.6 mm, I. D. 5 μm
Oven Temperature: 25°C
Eluent: Heptane/EtOH/ethanesulfonic acid = 95/5/0.1 (v/v/v)
Flow rate: 1 mL/min.
Detector: UV 210 nm

(3) LC conditions example 3: Method name LC-3 (used for analysis of optical purity)

LC: Agilent 1200
Column: CHIRALPAK IA
250 × 4.6 mm, I. D. 5 μm, two columns are connected
Oven Temperature: 25°C
Eluent: Heptane/EtOH/Trifluoroacetic acid = 96/4/0.1 (v/v/v) Flow rate: 1 mL/min.
Detector: UV 210 nm

(4) LC conditions example 4: Method name LC-4 (used for analysis of optical purity)

LC: Agilent 1200
Column: CHIRALPAK IA
250 × 4.6mm, I. D. 5 μm
Oven Temperature: 25°C
Eluent: Heptane/EtOH/Ethanesulfonic acid = 90/10/0.1 (v/v/v)
Flow rate: 1 mL/min.
Detector: UV 210 nm

[3] LC-MS

LC-MS: Waters 2695, MICROMASS QUATTRO MICRO API
Eluent: $CH_3CN$, 5 mM ammonium acetate aqueous solution
With respect to the analytical conditions, the analysis was carried out in the same method as LC-1 except Eluent.

Example 1: Synthesis of compound (11)

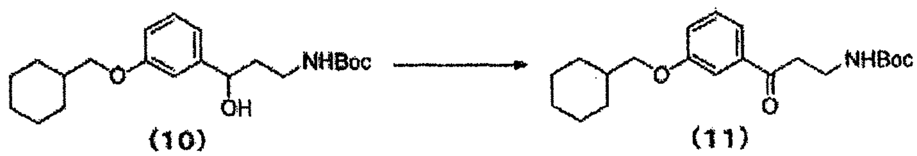

(10) (11)

The solution of 40 g of the compound (10) synthesized in accordance with the method described in Patent Document 1 in 400 g of toluene was cooled to 0°C. After completion of the cooling, 1.7 g of N-hydroxy-2-azaadamantane [AZADOL (registered trademark), sold by Wako Pure Chemical Industries, Ltd.] was added to the reaction solution. After completion of the addition, the reaction solution was stirred at the same temperature for 5 minutes. After completion of the stirring, 241 g of 5% by mass sodium hydrogen carbonate aqueous solution, and then 120 g of 14% by mass sodium hypochlorite aqueous solution were added dropwise to the reaction solution at the same temperature. After completion of the dropwise addition, the reaction solution was continuously stirred for 1 hour at 10°C to 15°C. After completion of the stirring, 1.7 g of N-hydroxy-2-azaadamantane [AZADOL (registered trademark), sold by Wako Pure Chemical Industries, Ltd.] was added to the reaction solution at 15°C to 20°C. After completion of the addition, 240 g of 5% by mass sodium hydrogen carbonate aqueous solution, and then 120 g of 14% by mass sodium hypochlorite aqueous solution were added dropwise to the reaction solution at 10°C to 15°C. After completion of the dropwise addition, the reaction solution was stirred at 10°C to 15°C for 2 hours. After completion of the stirring, the disappearance of the compound (10) being the starting material was ascertained by LC, and thereafter, 500 g of 10% by mass sodium thiosulfate aqueous

solution and then 220 g of 3.7% by mass hydrochloric acid aqueous solution were added dropwise at 7°C to 15°C to the reaction solution. After completion of the dropwise addition, the reaction solution was stirred at 15°C to 20°C for 30 minutes, and thereafter the organic phase was separated by a liquid separation operation. 420 g of toluene was added to the aqueous phase obtained after the liquid separation, and the resultant mixture was stirred for 10 minutes. Thereafter, the organic phase was separated by the liquid separation operation. The obtained organic phases were combined and 500 g of 10% by mass sodium hydrogen carbonate aqueous solution was added. The resultant mixture was stirred for 10 minutes and the mixture was separated. To the obtained organic phase, 500 g of water was added and the resultant mixture was stirred for 30 minutes. Thereafter, the mixture was separated and 500 g of 20% by mass sodium chloride aqueous solution was further added to the obtained organic phase and the resultant mixture was stirred for 10 minutes, followed by separating the mixture. To the obtained organic phase, 60 g of sodium sulfate was added and the resultant mixture was stirred for 30 minutes, followed by filtering the mixture. Thereafter, the filtrate was concentrated under reduced pressure to give 37.2 g of the crude product of the compound (11) as a brown oily substance.

Analysis conditions of LC at the time of ascertainment of starting material disappearance: LC-1

Purification of compound (11)

The crude product of the compound (11) obtained by the method described above was purified by column chromatography in accordance with the following conditions. 20.6 g of the compound (11) in which the area percentage obtained by the LC measurement is increased from 78% to 90% was obtained as a brown oily substance.

Column used: Hi-Flash Column, 40 μm, 60 A, 415 g
Gradient composition: n-hexane/ethyl acetate = 9/1 → 7/1 → 5/1 → 3/1 (volume ratio, the same applied hereafter).
Flow rate: 70 mL/min

The purified product obtained by column chromatography was purified by recrystallization. The conditions of the recrystallization will be described below. To 20.6 g of the column chromatography-purified compound (11) obtained by the method described above, 20.66 g of toluene and 122.4 g of n-hexane were added. After completion of the addition, the slurry solution of the compound (11) was heated to 45°C to completely dissolve the compound (11) in the solvent. When the temperature of the solution reached 42°C, 5 mg of a seed crystal of the compound (11) was added. After completion of the addition, occurrence of white turbidity in the solution was ascertained, and thereafter the solution was cooled to 2°C over 30 minutes. After completion of the cooling, the solution was stirred at 0°C to 2°C for 30 minutes. After completion of the stirring, the precipitated crystal in the solution was separated by filtration operation to give 13.9 g of the compound (11) as a white crystal. The yield from the compound (10) was 67.6% and the area percentage obtained by the LC measurement was 97.5%.
As the seed crystal of the compound (11), the crystal obtained after separately storing a part of the column chromatography-purified product in a frozen state at -15°C for 12 hours was used.
$^1$H-NMR of the compound (11) (500 MHz, ppm, in $CDCl_3$) δ: 1.00-1.10 (m, 2H), 1.19-1.25 (m, 1H), 1.25-1.35 (m, 2H), 1.40-1.50 (s, 9H), 1.68-1.75 (m, 1H), 1.75-1.85 (m, 3H), 1.85-1.90 (m, 2H), 3.16-3.21 (t, 2H), 3.51-3.58 (dd, 2H), 3.78-3.82 (d, 2H), 5.10-5.20 (t, 1H), 7.09-7.12 (dd, 1H), 7.34-7.38 (dd, 1H), 7.44-7.48 (s, 1H), 7.49-7.53 (d, 1H)
LC-MS (ESI +) m/z: 362 (M+H+)

LC conditions: LC-1

Example 2: Asymmetric reduction reaction of compound (11)

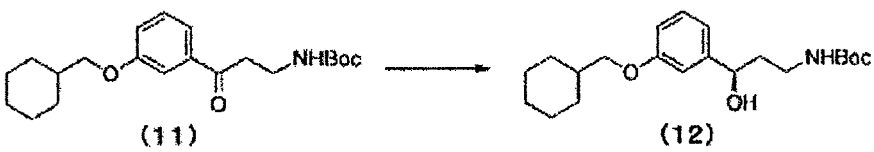

(11) (12)

To the solution of 0.20 g of the compound (11) in 1.0 g of ethanol and 1.0 g of 2-propanol, 6.5 mg of potassium tert-butoxide and 3.8 mg of (S)RUCY (registered trademark)-XylBINAP (sold by TAKASAGO INTERNATIONAL CORPORATION (compound of Formula (4)) were added. After completion of the addition, the inside of the vessel of the reaction solution was purged with hydrogen gas, and thereafter the reaction solution was stirred under a hydrogen

pressure of 0.5 MPa at a reaction temperature of 25°C for 1.5 hours. After completion of the stirring, the disappearance of the starting material in the reaction solution was ascertained by LC. After completion of the ascertainment, the reaction solution was concentrated under reduced pressure and diluted with acetonitrile using a 25 mL measuring flask. As a result of the quantitative analysis of the acetonitrile solution, the yield of the compound (12) was 99.7%. LC analysis under LC-2 conditions provides a stereoselectivity of the reduction reaction of 100:0.

Analysis conditions of LC at the time of ascertainment of starting material disappearance: LC-1

The quantitative analysis conditions of LC at the time of calculating the yield of the compound (12) were in accordance with the method described below.

Standard substance: The purified compound (10) [racemic substance of compound (12)] synthesized in accordance with the method described in Patent Document 1 was used as the standard substance.

Purification method: The solution of 6.06 g of the compound (10) in 7.0 g of toluene and 30 g of n-hexane was cooled to 0°C and stirred for 1 hour. After completion of the stirring, the precipitated white crystal in the solution was separated by a filtration operation. The obtained crystal was dried under reduced pressure to give 2.0 g of the compound (10) as a white crystal. The area percentage of the obtained crystal by the LC measurement was 99.2%.

LC conditions: LC-1

$^1$H-NMR of the compound (10) (500 MHz, ppm, in $CDCl_3$) $\delta$: 1.01-1.10 (m, 2H), 1.18-1.25 (m, 1H), 1.25-1.35 (m, 2H), 1.41 (s, 9H), 1.68-1.73 (m, 1H), 1.73-1.82 (m, 3H), 1.82-1.90 (m, 4H), 3.14-3.20 (m, 2H), 3.45-3.52 (m, 1H), 3.73-3.78 (d, 2H), 4.69-4.74 (m, 1H), 4.85-4.94 (t, 1H), 6.78-6.80 (d, 1H), 6.88-6.94 (m, 2H), 7.20-7.30 (dd, 1H)

LC-MS (ESI+) m/z: 386 (M+Na+)

The stereoselectivity of the compound (12) was calculated in accordance with the method described below. That is, as a result of the analysis of the compound (10) synthesized in accordance with the method described in Patent Document 1 by LC under the LC-2 conditions, the peaks having retention times of 10.9 minutes and 14.2 minutes were ascertained. For the compound (12) obtained by the reaction, only the peak having a retention time of 10.9 minutes was able to be ascertained.

The steric configuration of the obtained compound (12) was determined by deriving the compound (12) into the compound (1) in accordance with the method described below.
15 mL of the solution of the compound (12) obtained in Example 2 described above acetonitrile [as a result of the quantitative analysis described above, the compound (12) was contained in the solution in an amount of 60 mg] was concentrated under reduced pressure. The obtained compound (12) was dissolved into 1.0 g of methylene chloride, 0.61 g of water, and 0.97 g of trifluoroacetic acid were further added to this solution. After completion of the addition, the solution was stirred at 23°C for 1 hour. After completion of the stirring, the solution was heated to 50°C and continuously stirred at the same temperature for 2 hours. After completion of the stirring, the disappearance of the compound (12) and generation of the compound (1) were ascertained by LC under LC-1 conditions. Thereafter, the reaction liquid was concentrated under reduced pressure and the resultant residue was analyzed by LC under LC-2 conditions.

According to the description in Patent Document 1, the retention times of the compound (1) (R form) and the enantiomeric isomer of the compound (1) (S form) each analyzed by LC under LC-2 conditions are 29.485 minutes and 37.007 minutes, respectively. It was previously ascertained that analysis of the racemic form of the compound (1) synthesized in accordance with the method described in Patent Document 1 under the LC-2 conditions resulted in detection of the peaks of the compound (1) being the R form having a retention time of 34.8 minutes and the enantiomeric isomer of S form having a retention time of 40.7 minutes and the peak having a retention time of 34.8 minutes was the Compound (1). The configuration of the compound (1) was clarified to be (R) because the retention time of the compound (1) obtained in Example 2 was 34.9 minutes.

Example 3: Synthesis of compound (14)

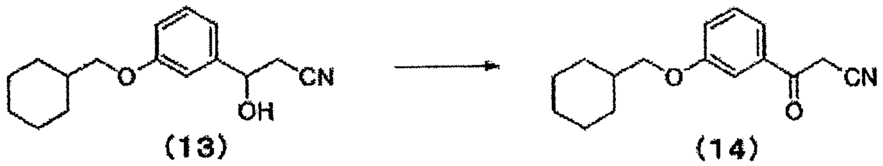

(13) (14)

To the solution of 30.0 g of the compound (13) synthesized in accordance with the method described in Patent Document 1 in 150 g of acetonitrile, 1.25 g of water, 403 mg of potassium 2-iodo-5-methylbenzenesulfonate and 60.7 g of OXONE (registered trademark, potassium monopersulfate compound salt) were sequentially added at 20°C to 25°C. After completion of the addition, the reaction solution was stirred at 74°C for 4.5 hours. After completion of the stirring, 1.0 g of water, 200 mg of potassium 2-iodo-5-methylbenzenesulfonate, and 7.2 g of OXONE were sequentially added at the same temperature to the reaction solution. After completion of the addition, the reaction solution was continuously stirred at the same temperature for 4 hours. After completion of the stirring, the disappearance of the compound (3) being the starting material was ascertained by LC, and the reaction solution was cooled to 20°C. Thereafter, insoluble substance in the reaction solution was separated by filtration with Celite. To the filtrate obtained after the filtration, 1.0 g of water, 200 mg of potassium 2-iodo-5-methylbenzenesulfonate and 60 g of OXONE were sequentially added. After completion of the addition, the reaction solution was stirred at 75°C for 3.5 hours. After completion of the stirring, the reaction solution was cooled to 20°C, and thereafter insoluble substance in the reaction solution was separated by filtration with Celite. The filtrate obtained after the filtration was concentrated under reduced pressure. To the obtained residue, 300 g of toluene, 60 g of water, and 2.0 g of sodium chloride were added and after the resultant mixture was stirred for 60 minutes, the aqueous phase was separated and disposed. To the obtained organic phase, sodium sulfate (30 g) was added and the resultant mixture was stirred at 20°C for 1 hour, followed by separating the mixture by filtration. The solvent in the obtained organic phase was distilled away under reduced pressure to give 31.5 g of the crude product of the compound (14) as a brown oily substance.

Analysis condition of LC at the time of ascertainment of starting material disappearance: LC-1

Purification of compound (14)

The crude product of the compound (14) obtained by the method described above was purified by column chromatography in accordance with the following conditions. 13.9 g of the compound (14) in which the area percentage obtained by the LC measurement was increased from 56% to 97% was obtained as a brown crystal.

Column used: Hi-Flash Column, 40 μm, 60 A, 265 g
Gradient composition: n-hexane/ethyl acetate =10/1 → 8/1 → 5/1 → 3/1 (flow rate 65 mL/min)

The compound (14) obtained by purifying with the column chromatography was further purified by recrystallization. Specifically, 13.3 g of the column chromatography-purified compound (14) obtained by the method described above was dissolved in 19.34 g of toluene at 60°C. After the dissolution, 18.62 g of n-hexane was added to the solution. After completion of the addition, the solution was cooled. When the internal temperature reached 40°C, 5 mg of the crystal of the compound (14) obtained after separately storing a part of the column chromatography purified product in a frozen state at -15°C for 12 hours was added as the seed crystal. After completion of the addition, the solution was continuously cooled. When the internal temperature reached 27°C, 15.47 g of toluene and 8.17 g of n-hexane were added. After completion of the addition, the solution was further continuously stirred at about 20°C for 1 hour. After completion of the stirring, the crystal precipitated in the solution was separated by filtration to give 7.74 g of the compound (14) as a white crystal. The yield from the compound (13) was 24% and the area percentage obtained by the LC measurement was 99.8%.

The obtained crystal was further purified with a preparative TLC. Specifically, first, 0.40 g of the crystal of the compound (14) obtained by the method described above was dissolved in 2 g of dichloromethane. Each half amount of the solution was charged to the two preparative TLC plates (Merck Kieselgel 60 F254 20 × 20 cm, thickness 2 mm) and development was carried out two times with hexane/ethyl acetate = 2/1 (volume ratio). 0.15 g of the compound (14) was obtained as a yellow solid from the part whose Rf value is about 0.6 to about 0.7. The yield from the crystal of the compound (14) was 38% and the area percentage of the obtained compound (14) obtained by the LC measurement was 100%.

$^1$H-NMR of compound (14) (500 MHz, ppm, in $CDCl_3$) δ: 1.06-1.12 (dt, 2H), 1.20-1.25 (m, 1H), 1.28-1.35 (m, 2H), 1.68-1.74 (m, 2H), 1.75-1.80 (m, 1H), 1.80-1.85 (m, 1H), 1.85-1.91 (m, 2H), 3.79-3.85 (d, 2H), 4.48 (s, 2H), 7.16-7.20 (m, 1H), 7.38-7.45 (m, 3H)

LC-MS (ESI +) m/z: 275 (M+NH4+)

Example 4: Asymmetric reduction reaction of compound (14)

To a mixed solution of 80.5 mg of triethylamine, 22.7 mg of formic acid, and 86.1 mg of N,N-dimethylformamide, 5.0 mg of RuCl[(R,R)-Tsdpen](p-cymene) (sold by KANTO CHEMICAL CO., INC.) (the compound of Formula (6)) was added at 20°C. After completion of the addition, 40.0 mg of the compound (14) was added to the reaction solution at the same temperature. After completion of the addition, the reaction solution was stirred at 30°C for 2 hours. After completion of the stirring, the disappearance of the compound (14) in the reaction solution was ascertained by LC under the LC-1 conditions, and thereafter the reaction liquid was diluted with acetonitrile using a 20 mL measuring flask. As a result of the quantitative analysis of the acetonitrile solution by LC, the yield of the compound (15) was 99.3%.

The solvent of 5 mL of the obtained acetonitrile solution was distilled away under reduced pressure, and thereafter the obtained compound (15) was diluted with 1.5 mL of the mobile phase in the LC-2 conditions. Analysis of the diluted compound (15) by LC under the LC-2 conditions provided the stereoselectivity of the reduction reaction of 98.95:1.05.

The yield of the compound (15) was calculated from the quantitative analysis by LC. LC-1 was used as the analysis conditions of LC. As the standard substance used in this quantitative analysis, the compound (13) [racemic substance of compound (15)] synthesized in accordance with the method described in Patent Document 1 purified with a silica gel column under the following conditions was used.

Column used: Hi-Flash Column, 40 μm, 60 A, 250 g
Gradient composition: n-hexane/ethyl acetate = 8/1 → 5/1 → 3/1 (flow rate 90 mL/min)

$^1$H-NMR of compound (15) (500 MHz, ppm, in $CDCl_3$) δ: 1.01-1.10 (m, 2H), 1.14-1.36 (m, 3H), 1.68-1.90 (m, 6H), 2.50-2.60 (m, 1H), 2.75-2.80 (m, 2H), 3.70-3.80 (m, 2H), 5.00 (t, 1H), 6.80-6.95 (m, 3H), 7.30-7.35 (m, 1H) LC-MS (ESI +) m/z: 277 (M+NH+)

The steric configuration of the obtained compound (15) was determined by deriving the compound (15) into the compound (1) in accordance with the method described below. That is, acetonitrile (5 mL) [10 mg of the compound (15) is contained in the acetonitrile solution] was concentrated under reduced pressure and the residue was dissolved in 0.6 g of tetrahydrofuran. The obtained solution was cooled. When the internal temperature reached 0°C, 93 mg of borane-dimethylsulfide complex was added. After completion of the addition, the reaction solution was heated to 70°C and stirred at the same temperature for 3 hours. After completion of the stirring, the disappearance of the compound (15) and generation of the compound (1) in the solution were ascertained by LC under LC-1 conditions. After completion of the ascertainment, the reaction solution was concentrated under reduced pressure. The obtained residue was analyzed in accordance with the analysis conditions described in Example 2. As a result, the compound (1) was clarified to be the (R) form because the retention time of the actually obtained compound (1) was 36.9 minutes.
(It was previously ascertained that analysis of the racemic form of the compound (1) synthesized in accordance with the method described in Patent Document 1 under the LC-2 conditions resulted in detection of the peaks having retention times of 36.9 minutes and 40.9 minutes).

Example 5: Synthesis of compound (2)

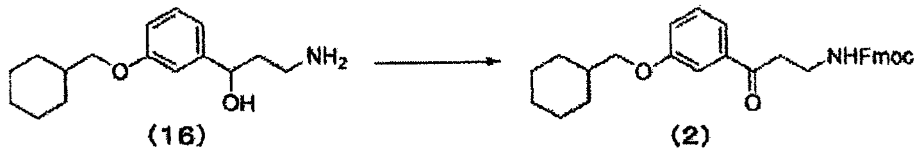

(16) (2)

To the solution of 3.04 g of the compound (16) synthesized in accordance with the method described in Patent Document 1 in 20 g of tetrahydrofuran, 10 g of water and 3.31 g of potassium carbonate were added. After completion of the addition, the reaction mixture was cooled to 5°C. Thereafter, the solution of 3.26 g of 9-fluorenylmethoxycarbonyl chloride in 20 g of tetrahydrofuran was added dropwise at about 5°C. After completion of the dropwise addition, the reaction mixture was stirred at room temperature for 1 hour. After completion of the stirring, the disappearance of the starting material in the reaction mixture was ascertained by LC. After ascertaining the disappearance of the starting material, 35 g of 1 mol/L hydrochloric acid aqueous solution was added dropwise to the reaction mixture at a temperature in a range of 0°C to 10°C. After completion of the dropwise addition, the reaction mixture was concentrated and thereafter

the residue was extracted two times with 50 g and 30 g of ethyl acetate. After combining the obtained organic phases, the combined organic phase was dehydrated and dried by adding 30 g of 20% by mass sodium chloride aqueous solution and then 10 g of sodium sulfate in this order. Thereafter, the solvent was distilled away under reduced pressure to give 6.00 g of a yellow oily substance. The obtained oily substance was dissolved in 60 g of toluene and 0.175 g of N-hydroxy-2-azaadamantane [AZADOL (registered trademark), sold by Wako Pure Chemical Industries, Ltd.] was added at 0°C. After completion of the addition, 25 g of 5% by mass sodium hydrogen carbonate aqueous solution, and then 12 g of 14% by mass sodium hypochlorite aqueous solution were added dropwise to the reaction solution at about 0°C. After completion of the dropwise addition, the reaction mixture was stirred at room temperature for 2 hours. Thereafter, 0.09 g of N-hydroxy-2-azaadamantane [AZADOL (registered trademark), sold by Wako Pure Chemical Industries, Ltd.], 12 g of 5% by mass sodium hydrogen carbonate aqueous solution, and then 6.15 g of 14% by mass sodium hypochlorite aqueous solution were added dropwise. After completion of the dropwise addition, the reaction mixture was stirred for 1 hour and thereafter the disappearance of the starting material in the reaction mixture was ascertained by LC. Thereafter, 70 g of 10% by mass sodium thiosulfate aqueous solution and then 22 g of 1 mol/L hydrochloric acid aqueous solution were added dropwise. After completion of the dropwise addition, the reaction mixture was stirred at room temperature for 20 minutes and thereafter extracted with toluene (50 g). After combining the obtained organic phases, the resultant organic phase was washed with 100 g of 10% by mass sodium hydrogen carbonate aqueous solution. Thereafter, the organic phase was dehydrated and dried by adding 100 g of 10% by mass sodium chloride aqueous solution and then 10 g of sodium sulfate in this order. Thereafter, the solvent was distilled away under reduced pressure to give 5.58 g of the crude product of the compound (2) being the target product as a brown oily substance.

Analysis conditions of LC at the time of ascertainment of starting material disappearance: LC-1

Purification of compound (2)

The crude product of the compound (2) obtained by the method described above was purified by column chromatography in accordance with the following condition (A). Subsequently, 2 g of the obtained compound (2) was purified again by column chromatography in accordance with the following condition (B). 1.1 g of the compound (2) was obtained as yellow oily substance by column chromatography purification carried out two times. The yield from the compound (16) was 20% and the area percentage of the obtained compound (2) obtained by the LC measurement was 88%.

Condition (A)

Column used: Hi-Flash Column, 40 $\mu$m, 60 Å, 120 g
Gradient composition: n-hexane/ethyl acetate = 88/12 $\rightarrow$ 85/15 (flow rate 60 mL/min)

Condition (B)

Column used: Hi-Flash Column, 40 $\mu$m, 60 Å, 120 g
Solvent composition: n-hexane/ethyl acetate = 9/1 (flow rate 60 mL/min)

$^1$H-NMR of compound (2) (500 MHz, ppm, in $CDCl_3$) $\delta$: 7.74 (d, J = 7.8 Hz, 2H), 7.57 (d, J = 7.8 Hz, 2H), 7.51 (d, J = 7.8 Hz, 1H), 7.47 (s-like, 1H), 7.37 (dd, J = 7.8, 7.8 Hz, 2H), 7.36 (dd, J = 7.8 Hz, 1H), 7.28 (dd, J = 7.8, 7.8 Hz, 2H), 7.11 (d-like, J = 7.8 Hz, 1H), 4.37 (d, J = 6.9 Hz, 2H), 4.19 (t, J = 6.9 Hz, 1H), 3.79 (d, J = 6.0 Hz, 2H), 3.62 (q, J = 5.7 Hz, 2H), 3.22 (t, J = 5.7 Hz, 2H), 1.88-1.86 (m, 2H), 1.84-1.76 (m, 3H), 1.72-1.70 (m, 1H), 1.34-1.27 (m, 2H), 1.25-1.18 (m, 1H), 1.10-1.03 (m, 2H)

LC-MS (ESI +) m/z: 501 (M+NH4+)

Example 6: Synthesis of compound (17)

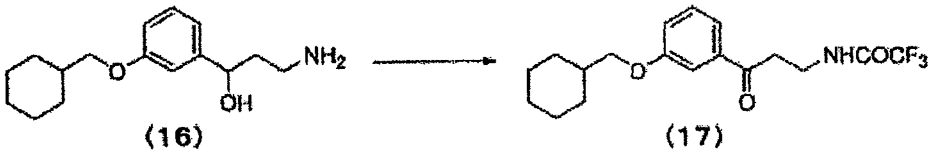

(16) (17)

To the solution of 3.07 g of the compound (16) synthesized in accordance with the method described in Patent Document 1 in 30 g of methylene chloride, 2.3 g of triethylamine was added and the resultant reaction mixture was cooled to about 0°C. To the reaction mixture, 2.59 g of trifluoroacetic anhydride was added dropwise at 10°C or lower. After completion of the dropwise addition, the reaction mixture was stirred at room temperature for 2 hours. After completion of the stirring, the disappearance of the starting material in the reaction mixture was ascertained by LC. After ascertaining the disappearance of the starting material, 10% by mass sodium hydrogen carbonate aqueous solution was added dropwise to the reaction liquid at 20°C or lower. The resultant mixture was stirred for 5 minutes and separated. The obtained aqueous phase was extracted with methylene chloride (20 g). After combining the obtained organic phases, the combined organic phase was dehydrated and dried by adding 10% by mass sodium chloride aqueous solution and then 5 g of magnesium sulfate in this order. Thereafter, the solvent was distilled away under reduced pressure to give 4.07 g of yellow oily substance. The solution of 2.14 g of the obtained oily substance in 21.4 g of toluene was cooled to 5°C and 0.09 g of N-hydroxy-2-azaadamantane [AZADOL (registered trademark), sold by Wako Pure Chemical Industries, Ltd.], 13 g of 5% by mass sodium hydrogen carbonate aqueous solution, and then 6.5 g of 14% by mass sodium hypochlorite aqueous solution were added dropwise. After completion of the dropwise addition, the reaction mixture was stirred at about 5°C, and the disappearance of the starting material in the reaction mixture was ascertained by LC 20 minutes later. After ascertaining the disappearance of the starting material, 30 g of 10% by mass sodium thiosulfate aqueous solution and then 10 g of 1 mol/L hydrochloric acid aqueous solution were added dropwise to the reaction mixture at about 10°C. After completion of the dropwise addition, the reaction liquid was stirred at room temperature for 30 minutes and then separated. The obtained aqueous phase was extracted with toluene (20 g). After combining the obtained organic phases, the resultant organic phase was washed with 20 g of 5% by mass sodium hydrogen carbonate aqueous solution. Thereafter, the organic phase was dehydrated and dried by adding 50 g of 10% by mass sodium chloride aqueous solution and then 10 g of magnesium sulfate in this order. Thereafter, the solvent was distilled away under reduced pressure to give 2.03 g of the crude product of the compound (17) being the target product.

Analysis condition of LC at the time of ascertainment of starting material disappearance: LC-1

Purification of compound (17)

The crude product of the compound (17) obtained by the method described above was purified by column chromatography in accordance with the following conditions. 1.45 g of the compound (17) whose area percentage obtained by the LC measurement was increased from 87% to 100% was obtained as a white crystal. In this case, the yield from the compound (16) was 35%.

Column used: Hi-Flash Column, 40 μm, 60 Å, 120 g
Solvent composition: n-hexane/ethyl acetate = 8/1 (flow rate 60 mL/min)

$^1$H-NMR of compound (17) (500 MHz, ppm, in $CDCl_3$) δ: 7.50 (d, J = 7.8 Hz, 1H), 7.45 (s-like 1H), 7.38 (dd, J = 7.8, 7.8 Hz, 1H), 7.15 (br, 1H), 7.14 (d, 1H, J = 7.8 Hz), 3.80 (d, J = 6. 0 Hz, 2H), 3.79 (d, J = 6. 0 Hz, 2H), 7.28 (d, J = 6. 0 Hz, 2H), 1.89-1. 86 (m, 2H), 1.85-1. 76 (m, 3H), 1.73-1. 70 (m, 1H), 1.34-1. 27 (m, 2H), 1.25-1. 17 (m, 1H), 1.10-1. 03 (m, 2H) LC-MS (ES +) m/z: 358 (M+H)

Example 7: Synthesis of compound (19)

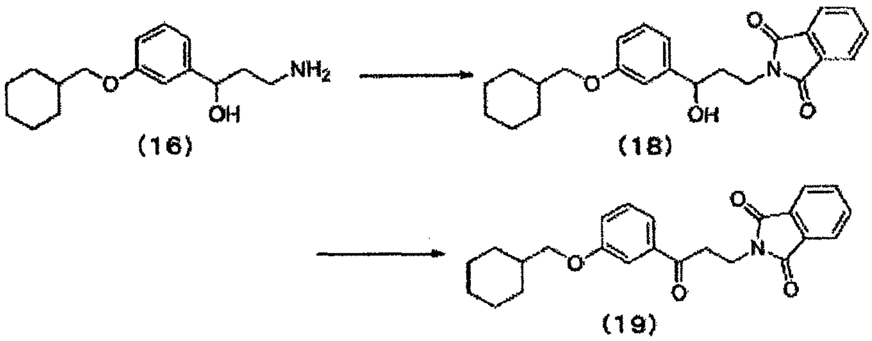

(16) (18)

(19)

To the solution of 3.06 g of the compound (16) synthesized in accordance with the method described in Patent Document 1 in 63 g of toluene, 1.88 g of phthalic anhydride and 1.70 g of N,N-diisopropylethylamine were added. After completion of the addition, the reaction mixture was refluxed for 2 hours and thereafter 0.13 g of phthalic anhydride and 0.56 g of N,N-diisopropylethylamine were added. After completion of the addition, the reaction mixture was continuously stirred for 1 hour and thereafter concentrated under reduced pressure. To the concentrated reaction liquid, 50 mL of ethyl acetate and 10 mL of 1 mol/L hydrochloric acid aqueous solution were added and the resultant mixture was separated. The obtained organic phase was washed with 50 g of 10% by mass sodium hydrogen carbonate aqueous solution. Thereafter, the organic phase was dehydrated and dried by adding 50 g of 15% by mass sodium chloride aqueous solution and then 10 g of sodium sulfate in this order. Thereafter, the solvent was distilled away under reduced pressure to give 5.2 g of the compound (18) being the target product as a yellow oily substance.

Purification of compound (18)

The crude product of the compound (18) obtained by the method described above was purified by column chromatography in accordance with the following conditions. 3.76 g of the compound (18) whose area percentage obtained by the LC measurement was increased to 99% was obtained.

Silica gel used: BW300SP, manufactured by Fuji Silysia Chemical Ltd., 50 g
Solvent composition: n-hexane/ethyl acetate = 2/1

To the solution of 3.37 g of the compound (18) obtained by the purification method described above in 33 g of toluene, 0.13 g of N-hydroxy-2-azaadamantane [AZADOL (registered trademark), sold by Wako Pure Chemical Industries, Ltd.], 18.3 g of 5% by mass sodium hydrogen carbonate aqueous solution, and 9.2 g of 14% by mass sodium hypochlorite aqueous solution were added. After completion of the addition, the reaction mixture was stirred at about 5°C for 1 hour. After completion of the stirring, the disappearance of the starting material in the reaction mixture was ascertained by LC. Thereafter, 40 g of 10% by mass sodium thiosulfate aqueous solution and 13 g of 1 mol/L hydrochloric acid aqueous solution were added dropwise at about 10°C. After completion of the dropwise addition, the reaction liquid was stirred at room temperature for 30 minutes and then extracted with toluene (20 g). After combining the obtained organic phases, the resultant organic phase was washed with 40 g of 10% by mass sodium hydrogen carbonate aqueous solution. Thereafter, the organic phase was dehydrated and dried by adding 50 g of 10% by mass sodium chloride aqueous solution and then 10 g of magnesium sulfate in this order. Thereafter, the solvent was distilled away under reduced pressure to give 2.57 g of the crude product of the compound (19) being the target product as a white crystal.

Analysis conditions of LC at the time of ascertainment of starting material disappearance: LC-1 1

Purification of compound (19)

The crude product of the compound (19) obtained by the method described above was purified by column chromatography in accordance with the following conditions. 1.1 g of the compound (19) whose area percentage obtained by the LC measurement was increased from 85% to 95% was obtained as yellow oily substance. In this case, the yield from the compound (16) was 24%.

Column used: Hi-Flash Column, 40 $\mu$m, 60 Å, 120 g
Solvent composition: n-hexane/ethyl acetate = 5/1 (flow rate 60 mL/min)

$^1$H-NMR of compound (19) (500 MHz, ppm, in $CDCl_3$) $\delta$: 7.85 (m, 2H), 7.72 (m, 2H), 7.49 (d-like, J = 7.8 Hz, 1H) 7.45 (s-like, 1H), 7.33 (dd, J = 7.8, 8. 1 Hz, 1H), 7.09 (d-like, J = 8. 1 Hz, 1H), 4. 14 (t, J = 7.5 Hz, 2H), 3.78 (d, J =

6. 6 Hz, 2H), 3.41 (t, J = 7.5 Hz, 2H), 1.87-1. 85 (m, 2H), 1.83-1. 74 (m, 3H), 1.71-1. 68 (m, 1H), 1.33-1. 26 (m, 2H), 1.24-1. 17 (m, 1H), 1.08-1. 02 (m, 2H) LC-MS (ES +) m/z: 392 (M+H)

Example 8: Synthesis of compound (20)

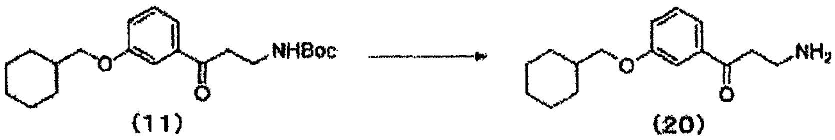

(11) (20)

To the solution of 2.99 g of the compound (11) synthesized in accordance with the method described in Example 1 in 21 g of dichloromethane, 12.01 g of trifluoroacetic acid was added. After completion of the addition, the reaction mixture was stirred at 45°C for 30 minutes. After completion of the stirring, the disappearance of the compound (11) being the starting material in the reaction mixture was ascertained by LC. After ascertaining the disappearance of the starting material, the reaction mixture was cooled to 10°C to 15°C. Thereafter, 20 g of 5% sodium hydrogen carbonate aqueous solution was added and the mixture was separated. The obtained aqueous phase was extracted two times with 20 g and 15 g of methylene chloride. After combining the obtained organic phases, the resultant organic phase was dehydrated and dried by adding 10 g of magnesium sulfate. Thereafter, the solvent was distilled away under reduced pressure to give the crude product of the compound (20) being the target product as a brown oily substance.

Analysis conditions of LC at the time of ascertainment of starting material disappearance: LC-1

Purification of compound (20)

The crude product of the compound (20) obtained by the method described above was purified by column chromatography in accordance with the following conditions. 1.1 g of the compound (20) whose area percentage obtained by the LC measurement is increased to 95% was obtained.

Silica gel used: Hi-Flash Column, 40 μm, 60 Å, 120 g
Solvent composition: n-hexane/ethyl acetate = 9/1 (flow rate 60 mL/min)

$^1$H-NMR of compound (20) (500 MHz, ppm, in $CDCl_3$) δ: 8. 21 (bs, 3H), 7.44 (d, J = 7.8 Hz, 1H), 7.39 (s-like, 1H), 7.31 (dd, J = 7.8, 7.8 Hz, 1H), 7.09 (d-like, J = 7.8 Hz, 1H), 3.74 (d, J = 6.6 Hz, 2H), 3.39 (m, 2H), 3.36 (m, 2H), 1.85-1. 83 (m, 2H), 1.79-1. 74 (m, 3H), 1.71-1. 68 (m, 1H), 1.32-1. 25 (m, 2H), 1.23-1. 15 (m, 1H), 1.07-1. 00 (m, 2H) LC-MS (ES +) m/z: 262 (M+H)

Example 9: Synthesis of compound (24)

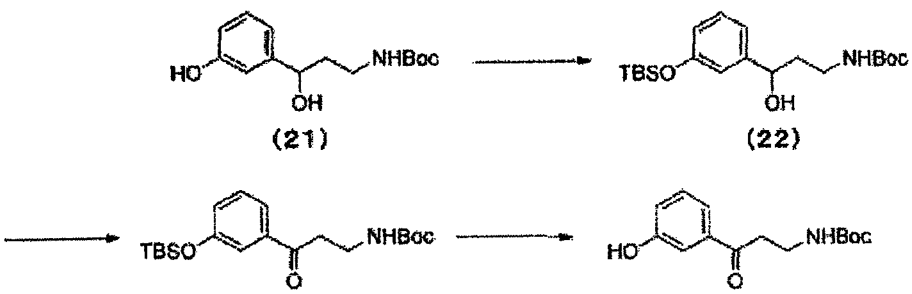

(21) (22) (23) (24)

To the solution of 17.7 g of the compound (21) synthesized in accordance with the method described in Patent Document 1 in 177.1 g of dichloromethane, 13.10 g of diazabicycloundecene and 11.01 g of tert-butyldimethylsilyl chloride (TBS-Cl) were added. After completion of the addition, the reaction mixture was stirred at room temperature for

1 hour. After completion of the stirring, the disappearance of the starting material in the reaction mixture was ascertained by LC. After ascertaining the disappearance of the starting material, 50 mL of water was added to the reaction liquid and the resultant mixture was separated. The obtained organic phase was washed with 50 mL of water. Thereafter, the organic phase was dehydrated and dried by adding 50 mL of 20% by mass salt solution and then 20 g of magnesium sulfate in this order. Thereafter, the solvent was distilled away under reduced pressure to give 28 g of the crude product of the compound (22).

To the solution of 25.0 g of the obtained crude product of the compound (22) in 250 g of toluene, 1.00 g of N-hydroxy-2-azaadamantane [AZADOL (registered trademark), sold by Wako Pure Chemical Industries, Ltd.], 143 g of 5% by mass sodium hydrogen carbonate aqueous solution, and 71.2 g of 14% by mass sodium hypochlorite aqueous solution were added. After completion of the addition, the reaction mixture was stirred at about room temperature for 3 hours. After completion of the stirring, the disappearance of the starting material in the reaction mixture was ascertained by LC. Thereafter, 50 g of 10% by mass sodium thiosulfate aqueous solution and 100 g of 3 mol/L hydrochloric acid aqueous solution were added dropwise at about 20°C. After completion of the dropwise addition, the reaction liquid was stirred at room temperature for 30 minutes and then separated. The obtained aqueous phase was extracted with 100 g of toluene. After combining the obtained organic phases, the resultant organic phase was washed with 50 g of 10% by mass sodium hydrogen carbonate aqueous solution. Thereafter, the solvent was distilled away under reduced pressure to give 24.1 g of the crude product of the compound (23).

Analysis conditions of LC at the time of ascertainment of starting material disappearance: LC-1

The crude product of the compound (23) was purified by column chromatography in accordance with the following conditions.

Column used: Hi-Flash Column, 40 μm, 60 Å, 120 g
Gradient composition: n-hexane/ethyl acetate = 96/4 → 80/20 (flow rate 70 mL/min)

The solution of 6.4 g of the obtained compound (23) by the purification method described above in 64 g of tetrahydrofuran was cooled to 0°C to 5°C. To the solution, 20 mL of tetrahydrofuran solution (1 M) of tetrabutylammonium fluoride was added dropwise. After completion of the dropwise addition, the reaction mixture was stirred at 0°C to 5°C for 1 hour. After completion of the stirring, the disappearance of the starting material in the reaction mixture was ascertained by LC under the LC-1 conditions. Thereafter, 12.8 g of ethyl acetate and 12.8 g of water were added to the reaction liquid and the resultant mixture was separated. The aqueous phase after the liquid separation was extracted with ethyl acetate (12.8 g). After combining the obtained organic phases, the solvent was distilled away under reduced pressure to give 4.0 g of the crude product of the compound (24).

The crude product of the compound (24) obtained by the method described above was purified by column chromatography in accordance with the following conditions to give 4.6 g of the compound (24). The yield from the compound (21) [three stages of TBS group formation, oxidation, and TBS group removal] was 26% and the area percentage of the compound (24) obtained by the LC measurement was 99%.

Silica gel used: Hi-Flash Column, 40 μm, 60 Å, 120 g
Gradient composition: n-hexane/ethyl acetate = 4/1 (flow rate 30 mL/min)

$^1$H-NMR of compound (23) (500 MHz, ppm, in $CDCl_3$) δ: 7.54 (d, J = 7.8 Hz, 1H), 7.41 (s-like, 1H), 7.32 (dd, J = 7.8, 7.8 Hz, 1H), 7.05 (d-like, J = 7.8 Hz, 1H), 5.15 (brs, 1H), 3.54 (q, J = 5.4 Hz, 2H), 3.17 (t, J = 5.4, 2H), 1.43 (s, 9H), 1.00 (s, 9H), 0. 22 (s, 6H) LC-MS(ES-)m/z:378 (M-H) $^1$H-NMR of compound (24) (500 MHz, ppm, in $CDCl_3$) δ: 9. 76 (s, 1H), 7.38 (d, J = 7.8 Hz, 1H), 7.32 (dd, J = 7.8, 7.8 Hz, 1H), 7.28 (s-like, 1H), 7.02 (d, 1H, J = 7.8 Hz), 6. 80 (t, J = 5.4 Hz, 1H), 3.25 (q, J = 5.4, 2H), 3.08 (t, J = 5.4, 2H), 1.36 (s, 9H) LC-MS (ES +) m/z: 266 (M+H)

Example 10: Synthesis of compound (29)

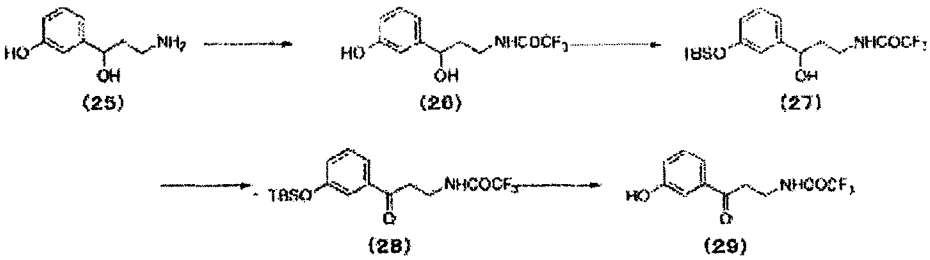

To the solution of 5.83 g of the compound (25) synthesized in accordance with the method described in Patent Document 1 in 100 g of dichloromethane, 6.09 g triethylamine was added and the reaction mixture was cooled to 5°C. After cooling, 7.01 g of trifluoroacetic anhydride was added dropwise at a temperature in a range of 3°C to 23°C. After completion of the dropwise addition, the reaction mixture was stirred at about 20°C for 3 hours. After completion of the reaction, 50 g of 20% by mass ammonium chloride aqueous solution was added to the reaction liquid and the resultant mixture was extracted with dichloromethane (50 g). After combining the obtained organic phases, the resultant organic phase was dehydrated and dried by adding 50 g of 20% by mass salt solution and then 10 g of magnesium sulfate in this order. Thereafter, the solvent was distilled away under reduced pressure to give 5.6 g of the crude product of the compound (26) as a brown oily substance.

To the solution of 2.00 g of the obtained compound (26) in 20 g of dichloromethane, 1.50 g of diazabicycloundecene and 1.26 g of tert-butyldimethylsilyl chloride were added. After completion of the addition, the reaction mixture was stirred at room temperature for 1 hour. After completion of the stirring, the disappearance of the starting material was ascertained under the LC-1 analysis conditions, 4 mL of water was added to the reaction liquid and the resultant mixture was separated. The obtained organic phase was washed with 4 mL of water. Thereafter, the organic phase was dehydrated and dried by adding 4 mL of 20% by mass salt solution and then 5 g of magnesium sulfate in this order. Thereafter, the solvent was distilled away under reduced pressure to give 2.7 g of the crude product of the compound (27).

To the solution of 2.9 g of the crude product of the compound (27) in 29.2 g of toluene, 120 mg of N-hydroxy-2-azaadamantane [AZADOL (registered trademark), sold by Wako Pure Chemical Industries, Ltd.], 16.78 g of 5% by mass sodium hydrogen carbonate aqueous solution, and 8.35 g of 14% by mass sodium hypochlorite aqueous solution were added. After completion of the addition, the reaction mixture was stirred at about room temperature for 1 hour. After completion of the stirring, the disappearance of the starting material in the reaction mixture was ascertained by LC. Thereafter, 15 g of 10% by mass sodium thiosulfate aqueous solution and 30 g of 3 mol/L hydrochloric acid aqueous solution were added at about 20°C, followed by extracting the resultant mixture with 10 g of toluene. The obtained organic phase was washed with 10 g of 10% by mass sodium hydrogen carbonate aqueous solution. The solvent of the obtained organic phase was distilled away under reduced pressure to give 2.8 g of the crude product of the compound (28).

The solution of 2.5 g of the compound (28) in 25 g of tetrahydrofuran was cooled to 0°C to 5°C. To the solution, 9.0 mL of tetrahydrofuran solution (1 M) of tetrabutylammonium fluoride was added dropwise and the resultant mixture was stirred at 0°C to 5°C for 1 hour. After completion of the stirring, the disappearance of the starting material was ascertained by LC. Thereafter, 5.0 g of ethyl acetate and 5.0 g of water were added to the reaction mixture and the resultant mixture was separated. The aqueous phase after the liquid separation was extracted with 5.0 g of ethyl acetate. After combining the obtained organic phases, the solvent was distilled away under reduced pressure to give 3.0 g of the crude product of the compound (29).

Analysis conditions of LC at the time of ascertainment of starting material disappearance: LC-1

The obtained crude product of the compound (29) was purified by column chromatography in accordance with the following conditions to give 2.1 g of the compound (29). The yield from the compound (25) was 41% and the area percentage obtained by the LC measurement was 95%.

Silica gel used: Hi-Flash Column, 40 μm, 60 Å, 50 g
Gradient composition: n-hexane/ethyl acetate = 1/1 (flow rate 30 mL/min)
$^1$H-NMR of the compound (29) (500 MHz, ppm, in DMSO) δ: 9.79 (s, 1H), 9.43 (bs, 1H), 7.41 (d, J = 7.8 Hz, 1H), 7.33 (dd, J = 7.8, 7.8 Hz, 1H), 7.31 (s-like, 1H), 7.03 (d, J = 7.8 Hz, 1H), 3.52 (q, 6.3 Hz, 2H), 3.25 (t, J = 6.3 Hz, 2H)

Example 11: Synthesis of compound (33)

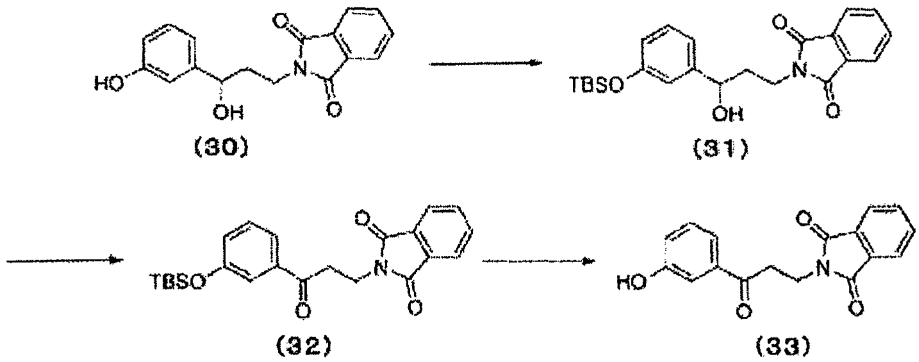

(30) (31) (32) (33)

To the solution of 1.90 g of the compound (30) synthesized in accordance with the method described in Patent Document 1 in 19.0 g of dichloromethane, 1.27 g of diazabicycloundecene and 1.05 g of tert-butyldimethylsilyl chloride were added and the resultant reaction mixture was stirred at room temperature for 1 hour. After completion of the stirring, the disappearance of the starting material was ascertained under the LC-1 conditions, and 4 mL of water was added to the reaction mixture, followed by separating the resultant mixture. The obtained organic phase was washed with 4 mL of water. Thereafter, the organic phase was dehydrated and dried by adding 4 mL of 20% by mass salt solution and then 5 g of magnesium sulfate in this order. Thereafter, the solvent was distilled away under reduced pressure to give 2.5 g of the crude product of the compound (31).

To the solution of 2.5 g of the obtained crude product of the compound (31) in 27.1 g of toluene, 100 mg of N-hydroxy-2-azaadamantane [AZADOL (registered trademark), sold by Wako Pure Chemical Industries, Ltd.], 14.3 g of 5% by mass sodium hydrogen carbonate aqueous solution, and 7.13 g of 14% by mass sodium hypochlorite aqueous solution were added. After completion of the addition, the reaction mixture was stirred at about room temperature for 1 hour. After completion of the stirring, the disappearance of the starting material in the reaction mixture was ascertained by LC under the LC-1 conditions. Thereafter, 15 g of 10% by mass sodium thiosulfate aqueous solution and 30 g of 3 mol/L hydrochloric acid aqueous solution were added dropwise at about 20°C. After completion of the dropwise addition, the resultant mixture was separated and the aqueous phase was extracted with 10 g of toluene. After combining the obtained organic phases, the resultant organic phase was washed with 10 g of 10% by mass sodium hydrogen carbonate aqueous solution. The solvent of the obtained organic phase was distilled away under reduced pressure to give 2.5 g of the crude product of the compound (32).

The solution of 2.5 g of the compound (32) in 25 g of tetrahydrofuran was cooled to 0°C to 5°C. To the reaction liquid, 8 mL of tetrahydrofuran solution (concentration 1 M) of tetrabutylammonium fluoride was added dropwise and the resultant mixture was stirred at 0°C to 5°C for 1 hour. After completion of the reaction, 5.0 g of ethyl acetate and 5.0 g of water were added to the reaction liquid and the resultant mixture was separated. The aqueous phase after the liquid separation was extracted with 5.0 g of ethyl acetate. The solvent of the obtained organic phase was distilled away under reduced pressure to give 3.0 g of the crude product of the compound (33).

The obtained crude product of the compound (33) was purified by column chromatography in accordance with the following conditions to give 2.0 g of the compound (33). The yield from the compound (30) [three stages of TBS group formation, oxidation, and TBS group removal] was 80% and the area percentage of the compound (33) obtained by the LC measurement was 95%.

Silica gel used: Hi-Flash Column, 40 μm, 60 Å, 50 g
Gradient composition: n-hexane/ethyl acetate = 1/1 (flow rate 100 mL/min)

$^1$H-NMR of compound (33) (500 MHz, ppm, in DMSO) δ: 9. 78 (s, 1H), 7.87-7.85 (m, 2H), 7.85-7.82 (m, 2H), 7.38 (d, J = 7.8 Hz, 1H), 7.30 (dd, J = 7.8,7.8 Hz, 1H), 7.28 (s-like, 1H), 7.01 (d, J = 7.8 Hz, 1H), 3.91 (t, J = 7.2 Hz, 2H), 3.37 (t, J = 7.2 Hz, 2H) LC-MS (ES +) m/z: 296 (M+H)

Example 12: Synthesis of compound (38)

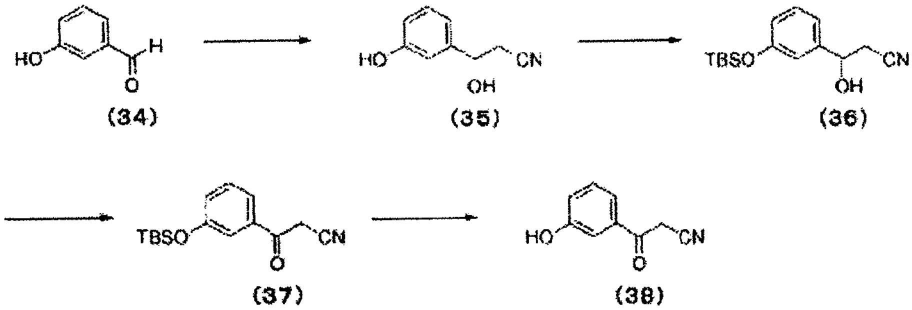

(34) (35) (36)

(37) (38)

Into a reaction flask, 213 g of potassium tert-butoxide and 1097 g of tetrahydrofuran were charged and the resultant mixture was stirred at -50°C for 1 hour. After completion of the stirring, 70 g of acetonitrile was added to the reaction mixture at -40°C to -50°C and the resultant mixture was stirred for 10 minutes. After completion of the stirring, the solution in which 86 g of 3-hydroxy benzaldehyde (34) was dissolved into 86 g of tetrahydrofuran was added dropwise to the reaction mixture at -30°C to -50°C, and the reaction liquid was stirred for 10 minutes. After completion of the stirring, the temperature of the reaction liquid was raised to 0°C and stirred for 1 hour. After completion of the stirring, 200 g of water was added to the reaction liquid and thereafter the solvent was distilled away under reduced pressure. 500 mL of ethyl acetate was added to the obtained residue and extraction operation was repeated two times. After combining the obtained organic phases, the combined organic phase was washed with 500 g of water. Thereafter, the organic phase was washed and dried by adding 400 mL of 20% by mass salt solution and then 30 g of magnesium sulfate in this order. Thereafter, the solvent was distilled away under reduced pressure to give 121 g of the crude product of the compound (35).

To the solution of 6.0 g of the obtained crude product of compound (35) in 50.0 g of N,N-dimethylformamide, 6.1 g of diazabicycloundecene was added and the resultant reaction mixture was stirred at room temperature. After completion of the stirring, 5.1 g of tert-butyldimethylsilyl chloride was added to the reaction mixture and the resultant reaction mixture was stirred at room temperature for 1 hour. After completion of the stirring, 4.6 g of diazabicycloundecene and 3.0 g of tert-butyldimethylsilyl chloride were added to the reaction mixture and the resultant reaction mixture was continuously stirred for 15 hours. After completion of the stirring, the solvent of the reaction liquid was distilled away under reduced pressure. Thereafter 50 mL of water and 100 mL of ethyl acetate were added and the resultant mixture was separated. The aqueous phase after the liquid separation was extracted with 30 L of ethyl acetate. After combining the obtained organic phases, the combined organic phase was washed with 50 mL of water. Thereafter, the organic phase was washed and dried by adding 50 mL of 20% by mass salt solution and then 10 g of magnesium sulfate in this order. Thereafter, the solvent was distilled away under reduced pressure to give 7.5 g of the crude product of the compound (36).

To the solution of 8.5 g of the obtained crude product of the compound (36) in 97.0 g of acetonitrile, 0.38 g of water, 1.2 g of potassium 2-iodo-5-methylbenzenesulfonate, and 20.2 g of OXONE (registered trademark, potassium monopersulfate compound salt) were added and the resultant reacting mixture was stirred at 80°C for 6 hours. After completion of the stirring, 100 mL of 20% by mass sodium thiosulfate aqueous solution was added to the reaction mixture and the resultant mixture was extracted with toluene (100 mL × 2). After combining the obtained organic phases, the combined organic phase was washed and dried by adding 100 mL of 20% by mass salt solution and then 10 g of magnesium sulfate in this order. Thereafter, the solvent was distilled away under reduced pressure to give 8.9 g of the crude product of the compound (37).

To the solution of 1.2 g of the obtained crude product of the compound (37) in 12.0 g of tetrahydrofuran, 4.8 g of 1 mol/L tetrahydrofuran solution of tetra-normal-butyl ammonium fluoride was added and the resultant reaction mixture was stirred at room temperature for 1 hour. After completion of the stirring, 10 mL of water and 10 mL of ethyl acetate were added to the reaction mixture and the resultant mixture was separated. The aqueous phase after the liquid separation was extracted with ethyl acetate (10 mL). After combining the obtained organic phases, the solvent was distilled away under reduced pressure to give 1.1 g of the crude product of the compound (38).

The obtained crude product of the compound (38) was purified by column chromatography in accordance with the following conditions to give 0.3 g of the compound (38). The area percentage of the compound (38) obtained by the LC measurement was 100%.

Silica gel used: Hi-Flash Column, 40 μm, 60 Å, 15 g
Gradient composition: n-hexane/ethyl acetate = 6/3 (flow rate 10 mL/min)

$^1$H-NMR of compound (37) (500 MHz, ppm, in DMSO) δ: 7.48 (d, J = 7.8 Hz, 1H), 7.38 (s-like, 1H), 7.38 (dd, J = 7.8, 7.8 Hz, 1H), 7.13 (d-like, J = 7.8 Hz, 1H), 4.05 (s, 2H), 1.00 (s, 9H), 0.23 (s, 6H)

LC-MS (ES +) m/z: 276 (M+H)

$^1$H-NMR of compound (38) (500 MHz, ppm, in DMSO) δ: 9. 92 (s, 1H), 7.38 (d, J = 7.8 Hz, 1H), 7.35 (dd, J = 7.8,7.8 Hz, 1H), 7.28 (s, 1H), 7.09 (d-like, J = 7.8 Hz, 1H), 4. 7 (s, 2H) LC-MS (ES -) m/z: 160 (M-H)

Example 13: Synthesis of compound (39)

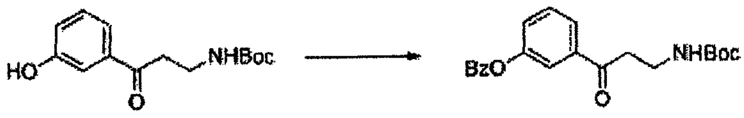

(24) (39)

To the solution of 0.3 g of the compound (24) in 3.6 g of dichloromethane, 0.2 g of pyridine and 0.2 g of benzoyl chloride were added and the resultant reaction mixture was stirred at room temperature for 5 hours. After completion of the stirring, 10 mL of 20% by mass ammonium chloride aqueous solution and 10 mL of ethyl acetate were added to the reaction mixture and the resultant mixture was separated. The aqueous phase after the liquid separation was extracted with ethyl acetate (10 mL × 2). After combining the obtained organic phases, the resultant organic phase was washed with 10 mL of water. Thereafter, the organic phase was dehydrated and dried by adding 10 mL of 15% by mass salt solution and then 2.1 g of magnesium sulfate in this order. Thereafter, the solvent was distilled away under reduced pressure to give 0.5 g of the crude product of the compound (39).

The obtained crude product of the compound (39) was purified by column chromatography in accordance with the following conditions to give 0.4 g of the compound (39). The area percentage of the compound (39) obtained by the LC measurement was 100%.

Silica gel used: Hi-Flash Column, 40 μm, 60 Å, 15 g
Gradient composition: n-hexane/ethyl acetate = 100/0 (flow rate 15 mL/min)

$^1$H-NMR of compound (23) (500 MHz, ppm, in $CDCl_3$) δ: 8. 21 (d, J = 7.8 Hz, 2H), 7.87 (d, J = 7.8 Hz, 1H), 7.81 (s-like, 1H), 7.66 (t, 1H, J = 7.8 Hz), 7.55 (dd, J = 7.8 Hz, 1H), 7.53 (dd, J = 7.8, 7.8 Hz, 2H), 7.46 (d, J = 7.8, 1H), 5.14 (brs, 1H), 3.56 (q, J = 5.4, 2H), 3.22 (t, J = 5.4, 2H), 1.43 (s, 9H)

LC-MS (ES+) m/z: 370 (M+H)

Example 14: Synthesis of compound (40)

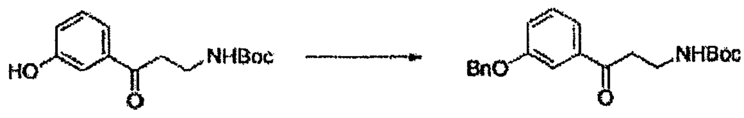

(24) (40)

To the solution of 0.3 g of the compound (24) in 3.0 g of N,N-dimethylformamide, 0.3 g of potassium carbonate and 0.3 g of benzyl chloride were added and the obtained reaction mixture was stirred at 60°C for 5 hours. After completion of the stirring, the reaction mixture was cooled to 0°C. Thereafter, 20 mL of 20% by mass ammonium chloride aqueous solution and 10 mL of ethyl acetate were added and the resultant mixture was separated. The aqueous phase after the liquid separation was extracted with ethyl acetate (10 mL × 2). After combining the obtained organic phases, the combined organic phase was washed with 10 mL of water. Thereafter, the organic phase was dehydrated and dried by adding 10 mL of 15% by mass salt solution and then 5.0 g of magnesium sulfate in this order. Thereafter, the solvent was distilled away under reduced pressure to give 0.5 g of the crude product of the compound (40).

The obtained crude product of the compound (40) was purified by column chromatography in accordance with the following conditions to give 0.3 g of the compound (40). The area percentage of the compound (40) obtained by the LC measurement was 100%.

Silica gel used: Hi-Flash Column, 40 μm, 60 Å, 15 g
Gradient composition: n-hexane/ethyl acetate = 90/10 → 4/1 (flow rate 15 mL/min)

$^1$H-NMR of compound (40) (500 MHz, ppm, in $CDCl_3$) δ: 7.57 (s-like, 1H), 7.55 (d, J = 7.8 Hz, 1H), 7.44 (d, J = 7.8 Hz, 2H), 7.40 (dd, J = 7.8, 7.8 Hz, 2H), 7.38 (dd, J = 7.8, 7.8 Hz, 1H), 7.34 (t, J = 7.8 Hz, 1H), 7.19 (d, J = 7.8, 1H), 5.12 (brs, 1H), 5.12 (s, 2H), 3.54 (q, J = 6. 0,2H), 3.18 (t, J = 6. 0,2H), 1.43 (s, 9H)

LC-MS (ES+) m/z: 356 (M+H)

Example 15: Synthesis of compound (41)

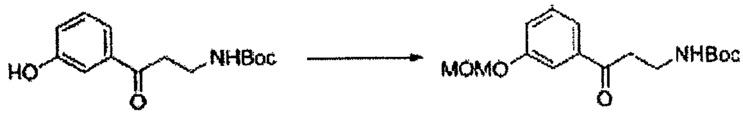

(24) (41)

To the solution of 0.5 g of the compound (24) in 8.5 g of dichloromethane, 0.5 g of diisopropylethylamine and 0.2 g of methoxymethyl chloride were added and the resultant reaction mixture was stirred at room temperature for 5 hours. After completion of the stirring, the reaction mixture was cooled to 0°C. Thereafter, 20 mL of 20% by mass ammonium chloride aqueous solution and 30 mL of ethyl acetate were added and the resultant mixture was separated. The obtained organic phase was washed with 20 mL of water. Thereafter, the organic phase was dehydrated and dried by adding 20 mL of 15% by mass salt solution. Thereafter, the solvent was distilled away under reduced pressure to give 0.7 g of the crude product of the compound (41).

The obtained crude product of the compound (40) was purified by column chromatography in accordance with the following conditions to give 0.4 g of the compound (41). The area percentage of the compound (41) obtained by the LC measurement was 100%.

Silica gel used: Hi-Flash Column, 40 μm, 60 Å, 15 g
Gradient composition: n-hexane/ethyl acetate = 100/0 → 4/1 (flow rate 15 mL/min)

$^1$H-NMR of compound (41) (500 MHz, ppm, in $CDCl_3$) δ: 7.62 (s-like, 1H), 7.59 (d, J = 7.8 Hz, 1H), 7.38 (dd, J = 7.8, 7.8 Hz, 1H), 7.25 (d, 1H, J = 7.8 Hz), 5.22 (s, 2H), 5.18 (brs, 1H), 3.54 (q, J = 5.4, 2H), 3.48 (s, 3H), 3.19 (t, J = 5.4, 2H), 1.42 (s, 9H) LC-MS (ES+) m/z: 327 (M+NH4)

Example 16: Asymmetric reduction reaction using catalyst (4), that is, (S)-RUCY-XylBINAP as catalyst

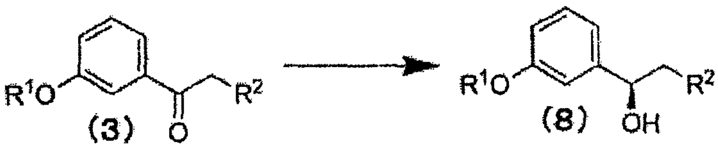

(3) (8)

To the solution of 100 mg of the ketone (3) in ethanol [5 times in mass relative to ketone (3) was used] and 2-propanol [5 times in mass relative to ketone (3) was used], potassium tert-butoxide [0.2 times in mole relative to ketone (3) was used] and catalyst (4), that is, (S)-RUCY (registered trademark)-XylBINAP (sold by TAKASAGO INTERNATIONAL CORPORATION) [0.01 times in mole relative to ketone (3) was used] were added. After completion of the addition, the inside of the vessel of the reaction solution was purged with hydrogen gas, and thereafter the reaction solution was reacted under a hydrogen pressure of 0.5 MPa at a reaction temperature of 25°.

After ascertaining the disappearance of the starting material by LC analysis, the reaction liquid was diluted with acetonitrile using a 25 mL measuring flask. The quantitative analysis of the acetonitrile diluted solution was carried out to determine the yield of the compound (8). As the standard substance used in the quantitative analysis of the compound (8), the compound obtained by purifying the racemic form of the compound (8) obtained at the time of synthesizing the compound (3) was used.

Analysis condition of LC at the time of ascertainment of starting material disappearance: LC-1

Conditions at the time of quantitative analysis: LC-1

The starting materials to be used, the yields of the reduction reaction, and the optical purities of the compound (8) being the product are listed in the following table.

Table 1

| Example No. | Starting material No. | Yield (%) | Optical purity (%ee) |
|---|---|---|---|
| 16-1 | (17) | 100 | 100 |
| 16-2 | (19) | 45 | 88 |
| 16-3 | (2) | 14 | 100 |
| 16-4 | (20) | 0 | - |
| 16-5 | (24) | 0 | - |
| 16-6 | (29) | 0 | - |
| 16-7 | (33) | 0 | - |
| 16-8 | (14) | 0 | - |
| 16-9 | (38) | 0 | - |
| 16-10 | (39) | 0 | - |
| 16-11 | (40) | 100 | 95 |
| 16-12 | (41) | 100 | 93 |
| 16-13 | (23) | 100 | 99 |

In the above table, the reactions were not progressed and the starting materials were recovered in Examples 16-4 to 16-10. In the columns of Yield of Example 16-11 to 16-13, reaction conversion rates are listed.

The reaction conversion rate was calculated based on [LC area value of compound (8)]/[Sum of LC area values of compounds (8) and (3)]×100%.

The optical purities of the reaction products in Example 16-1 to 16-3 were obtained by deriving each reaction product into the compound (1) in accordance with the methods described below and thereafter calculating the optical purities in accordance with the analytical conditions described in Example 2.

Example 16-1: 5 mL of the acetonitrile diluted solution used for the quantitative analysis was concentrated, and 1 g of methanol, 0.11 g of potassium carbonate, and 0.26 g of water were added to the obtained residue, followed by stirring the resultant mixture at room temperature. After completion of the stirring, the reaction liquid was concentrated under reduced pressure and the obtained residue was used for the analysis of the optical purity.

Example 16-2: 5 mL of the acetonitrile diluted solution used for the quantitative analysis was concentrated, and 1 g of ethanol and 0.10 g of hydrazine monohydrate were added to the obtained residue, followed by stirring the resultant mixture at 80°C for 1 hour. After completion of the stirring, the reaction liquid was concentrated under reduced pressure and the obtained residue was used for the analysis of the optical purity.

Example 16-3: 10 mL of the acetonitrile diluted solution used for the quantitative analysis was concentrated, and 0.5 g of dimethylformamide and 0.25 g of morpholine were added to the obtained residue, followed by stirring the resultant mixture at room temperature for 0.5 hour. After completion of the stirring, the reaction liquid was concentrated under reduced pressure and the obtained residue was used for the analysis of the optical purity.

The optical purities of the reaction products in Example 16-11 to 16-13 were obtained by deriving each reaction product into the compound [(R)-21] in accordance with the methods described below and thereafter calculating the optical purities in accordance with the analytical conditions of LC-3.

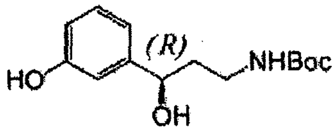

[(R)-21]

Example 16-11: 10 mL of the acetonitrile diluted solution used for the quantitative analysis was concentrated, and 0.5 g of ethanol and 0.1 g of 10% by mass palladium carbon were added to the obtained residue. After completion of the addition, the reaction liquid was stirred under hydrogen gas atmosphere at 40°C for 0.5 hour. After completion of the stirring, the reaction liquid was concentrated under reduced pressure and the obtained residue was used for the analysis of the optical purity.

Example 16-12: 10 mL of the acetonitrile diluted solution used for the quantitative analysis was concentrated, and 0.5 g of methanol and 0.1 g of hydrochloric acid (1 M) were added to the obtained residue. After completion of the addition, the reaction liquid was stirred at 60°C for 2 hours. After completion of the stirring, the reaction liquid was concentrated under reduced pressure and the obtained residue was used for the analysis of the optical purity.

Example 16-13: 10 mL of the acetonitrile diluted solution used for the quantitative analysis was concentrated, and 0.5 g of tetrahydrofuran and 0.1 g of 1 mol/L tetrahydrofuran solution of tetra-normal-butylammonium fluoride were added to the obtained residue, followed by stirring the resultant mixture at room temperature for 2 hours. The reaction liquid was concentrated under reduced pressure and the obtained residue was used for the analysis of the optical purity.

Example (17): Asymmetric reduction reaction using catalyst (5), that is, $RuCl_2$[(S)-XylBINAP][(S)-daipen] as catalyst

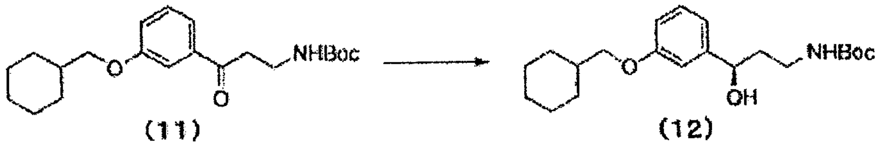

(11) (12)

To the solution of 0.10 g of the compound (11) in 0.51 g of 2-propanol and 0.53 g of ethanol, 6.4 mg of potassium tert-butoxide and 4.0 mg of catalyst (5), that is, $RuCl_2$[(S)-xylbinap][(S)-daipen] (sold by TAKASAGO INTERNATIONAL CORPORATION) were added. After completion of the addition, the inside of the vessel of the reaction solution was purged with hydrogen gas, and thereafter the reaction solution was stirred under a hydrogen pressure of 0.5 MPa at a reaction temperature of 25°C for 1 hour. After completion of the stirring, the disappearance of the starting material in the reaction solution was ascertained by LC. After completion of the ascertainment, the reaction solution was concentrated under reduced pressure and diluted with acetonitrile using a 25 mL measuring flask. As a result of the quantitative analysis of the acetonitrile solution, the yield of the compound (12) was 98.6%. LC analysis was performed under the LC quantitative analysis conditions described in Example 2 to determine the yield. LC analysis under LC-2 conditions provides a stereoselectivity of the reduction reaction of 100:0. The stereoselectivity was determined in accordance with the method described in Example 2.

Analysis conditions of LC at the time of ascertainment of starting material disappearance: LC-1

Example 18: Asymmetric reduction reaction using catalyst (4)

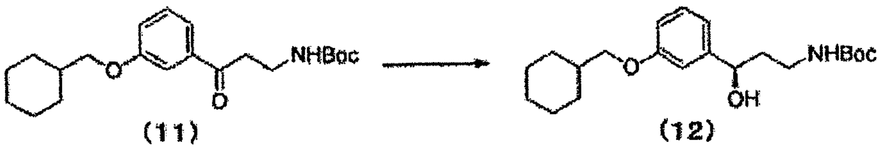

(11) (12)

To the solution of 0.30 g of the compound (11) in 6.0 mL of 2-propanol. and 6.0 mL of ethanol, 4.9 mg of potassium tert-butoxide and 1.0 mg of catalyst (4), that is, (S)-RUCY (registered trademark)-XylBINAP (sold by TAKASAGO INTERNATIONAL CORPORATION were added. After completion of the addition, the inside of the vessel of the reaction solution was replaced with hydrogen gas, and thereafter the reaction solution was stirred under a hydrogen pressure of 0.9 MPa at a reaction temperature of 25°C for 2 hours. After completion of the stirring, the disappearance

of the starting material in the reaction solution was ascertained by LC. After completion of the ascertainment, the reaction solution was concentrated under reduced pressure and diluted with acetonitrile using a 25 mL measuring flask. LC analysis under LC-2 conditions provides a stereoselectivity of the reduction reaction of 100:0. The stereoselectivity was determined in accordance with the method described in Example 2.

Analysis conditions of LC at the time of ascertainment of starting material disappearance: LC-1

Example 19: Asymmetric reduction reaction using catalyst (6)

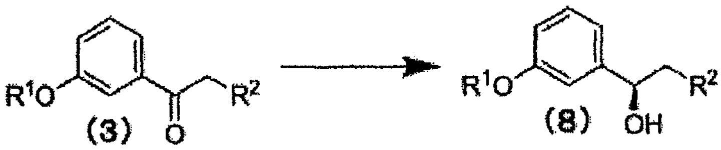

To the solution of 100 mg of the ketone (3) in dimethylformamide [2 times in mass relative to ketone (3) was used], Formic acid [3.1 times in mole relative to ketone (3) was used], triethylamine [5.2 times in mole relative to ketone (3) was used], and 5.0 mg of RuCl[(R,R)-Tsdpen](p-cymene) (sold by KANTO CHEMICAL CO., INC.) were added. After completion of the addition, the reaction solution was reacted at a temperature of 35°C for 3 to 6 hours.

After ascertaining the disappearance of the starting material by LC analysis, the reaction liquid was diluted with acetonitrile using a 25 mL measuring flask. The quantitative analysis of the acetonitrile diluted solution was carried out to determine the yield of the compound (8). As the standard substance used in the quantitative analysis of the compound (8), the compound obtained by purifying the racemic form of the compound (8) obtained at the time of synthesizing the compound (3) was used.

Analysis condition of LC at the time of ascertainment of starting material disappearance: LC-1

Conditions at the time of quantitative analysis: LC-1

The starting materials to be used, the yields of the reduction reaction, and the optical purities of the compound (8) being the product are listed in the following table.

Table 2

| Example No. | Starting material No. | Yield (%) | Optical purity (%ee) |
|---|---|---|---|
| 19-1 | (11) | 99 | 91 |
| 19-2 | (17) | 100 | 96 |
| 19-3 | (19) | 84 | 87 |
| 19-4 | (2) | 0 | - |
| 19-5 | (20) | 46 | 96 |
| 19-6 | (24) | 94 | 97 |
| 19-7 | (29) | 91 | 96 |
| 19-8 | (33) | 92 | 96 |
| 19-9 | (38) | 100 | 99 |
| 19-10 | (40) | 98 | 84 |
| 19-11 | (39) | 100 | 90 |
| 19-12 | (41) | 99 | 85 |
| 19-13 | (37) | 100 | 98 |
| 19-14 | (23) | 100 | 90 |

In the above table, the compound (2) was decomposed in Example 19-4 and thus the reduction reaction product was not obtained. In the columns of Yield of Example 19-9 to 19-14, reaction conversion rates are listed.

The reaction conversion rate was calculated based on [LC area value of compound (8)]/[Sum of LC area values of compounds (8) and (3)]$\times$100%.

The optical purities of the reaction products in Example 19-1, 19-5, and 19-6 were obtained by concentrating 5 mL of the acetonitrile diluted solution used for the quantitative analysis, analyzing the obtained residue in accordance with the analysis conditions described in Example 2, and calculating.

The optical purities of the reaction products in Example 19-2 and 19-3 were obtained by deriving each reaction product into the compound (1) in accordance with the methods described below and thereafter calculating the optical purities in accordance with the analytical conditions described in Example 2.

Example 19-2: 5 mL of the acetonitrile diluted solution used for the quantitative analysis was concentrated, and 2 g of methanol, 0.10 g of potassium carbonate, and 0.5 g of water were added to the obtained residue, followed by stirring the resultant mixture at room temperature for 0.5 hour. After completion of the stirring, the reaction liquid was concentrated under reduced pressure and the obtained residue was used for the analysis of the optical purity.

Example 19-3: 5 mL of the acetonitrile diluted solution used for the quantitative analysis was concentrated, and 1.0 g of ethanol and 0.1 g of hydrazine monohydrate were added to the obtained residue, followed by stirring the resultant mixture at 80°C for 1 hour. After completion of the stirring, the reaction liquid was concentrated under reduced pressure and the obtained residue was used for the analysis of the optical purity.

The optical purities of the reaction products in Example 19-7 and 19-8 were obtained by deriving each reaction product into the compound [(R)-25] in accordance with the methods described below and thereafter calculating the optical purities in accordance with the analytical conditions described in Example 2.

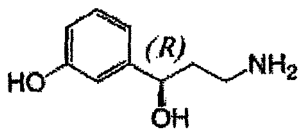

[(R)-25]

Example 19-7: 5 mL of the acetonitrile diluted solution used for the quantitative analysis was concentrated, and 2.0 g of methanol, 0.1 g of potassium carbonate, and 0.5 g of water were added to the obtained residue, followed by stirring the resultant mixture at room temperature for 2 hours. After completion of the stirring, the reaction liquid was concentrated under reduced pressure and the obtained residue was used for the analysis of the optical purity.

Example 19-8: 5 mL of the acetonitrile diluted solution used for the quantitative analysis was concentrated, and 2.0 g of ethanol and 0.1 g of hydrazine monohydrate were added to the obtained residue, followed by stirring the resultant mixture at 80°C for 80 minutes. After completion of the stirring, the reaction liquid was concentrated under reduced pressure and the obtained residue was used for the analysis of the optical purity.

The optical purity of the reaction products in Example 19-9 was obtained by concentrating 5 mL of the acetonitrile diluted solution used for the quantitative analysis, and calculating the optical purity of the obtained residue in accordance with the analytical conditions of LC-4.

The optical purities of the reaction products in Example 19-10, 19-11, 19-12 and 19-14 were obtained by deriving each reaction product into the compound [(R)-21] in accordance with the methods described below and thereafter calculating the optical purities in accordance with the analytical conditions of LC-3.

Example 19-10: 5 mL of the acetonitrile diluted solution used for the quantitative analysis was concentrated, and 0.5 g of ethanol and 10 mg of palladium carbon were added to the obtained residue, followed by stirring the reaction mixture under hydrogen atmosphere at room temperature for 6 hours. After completion of the stirring, the 10% by mass palladium carbon was separated by filtration and the obtained filtrate was concentrated under reduced pressure, followed by using the obtained residue for the analysis of the optical purity.

Example 19-11: 5 mL of the acetonitrile diluted solution used for the quantitative analysis was concentrated, and 0.5 g of methanol and 0.1 g of 1% by mass sodium hydroxide aqueous solution were added to the obtained residue, followed by stirring the resultant mixture at room temperature for 2 hours. After completion of the stirring, the reaction liquid was concentrated under reduced pressure and the obtained residue was used for the analysis of the optical purity.

Example 19-12: 5 mL of the acetonitrile diluted solution used for the quantitative analysis was concentrated, and 0.5 g of methanol and 50 mg of hydrochloric acid (1 M) were added to the obtained residue, followed by stirring the resultant mixture at room temperature for 2 hours. After completion of the stirring, the reaction liquid was concentrated under reduced pressure and the obtained residue was used for the analysis of the optical purity.

Example 19-14: 10 mL of the acetonitrile diluted solution used for the quantitative analysis was concentrated, and 0.5 g of tetrahydrofuran and 0.1 g of 1 Mol/L tetrabutylammonium fluoride were added to the obtained residue,

followed by stirring the resultant mixture at room temperature for 2 hours. After completion of the stirring, the reaction liquid was concentrated under reduced pressure and the obtained residue was used for the analysis of the optical purity.

The optical purity of the reaction products in Example 19-13 was obtained by deriving the reaction product into the compound [(R)-35] in accordance with the methods described below and thereafter calculating the optical purities in accordance with the analytical conditions of LC-4.

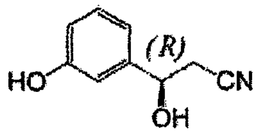

[(R)-35]

Example 19-13: 5 mL of the acetonitrile diluted solution used for the quantitative analysis was concentrated, and 1.0 g of tetrahydrofuran and 50 mg of tetrabutylammonium fluoride were added to the obtained residue, followed by stirring the resultant mixture at room temperature for 7 hours. After completion of the stirring, the reaction liquid was concentrated under reduced pressure and the obtained residue was used for the analysis of the optical purity.

Example 20: Asymmetric reduction reaction using catalyst (7), that is, (S)-5,5-diphenyl-2-methyl-3,4-propano-1,3,2-oxazaborolidine as catalyst

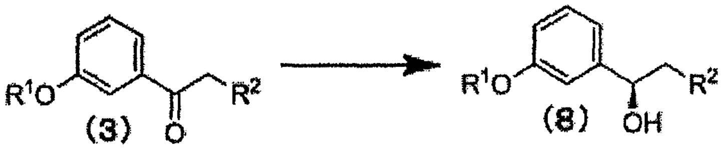

To the solution of (S)-5,5-diphenyl-2-methyl-3,4-propano-1,3,2-oxazaborolidine [0.3 times in mole relative to ketone (3) was used] in methylene chloride [10 times in mass relative to ketone (3) was used], Borane-dimethylsulfide complex [1.5 times in mole relative to ketone (3) was used] was added, and thereafter the reaction liquid was cooled to -50°C. After completion of the cooling, the solution of 100 mg of the ketone (3) in methylene chloride [10 times in mass relative to ketone (3) was used] was added dropwise to the reaction liquid and the resultant reaction liquid was reacted at -50°C for 2 hours.

After ascertaining the disappearance of the starting material by LC analysis, the reaction liquid was diluted with acetonitrile using a 25 mL measuring flask. The quantitative analysis of the acetonitrile diluted solution was carried out to calculate the yield of the compound (8). As the standard substance used in the quantitative analysis of the compound (8), the compound obtained by purifying the racemic form of the compound (8) obtained at the time of synthesizing the compound (3) was used.

Analysis conditions of LC at the time of ascertainment of starting material disappearance: LC-1

Conditions at the time of quantitative analysis: LC-1

The starting materials to be used, the yields of the reduction reaction, and the optical purities of the compound (8) being the product are listed in the following table.

Table 3

| Example No. | Starting material No. | Yield (%) | Optical purity (%ee) |
|---|---|---|---|
| 20-1 | (11) | 27 | 82 |
| 20-2 | (17) | 79 | 90 |
| 20-3 | (20) | 72 | 4 |
| 20-4 | (19) | 65 | 74 |
| 20-5 | (2) | 45 | 40 |
| 20-6 | (14) | 83 | 7 |
| 20-7 | (24) | 12 | 39 |

(continued)

| Example No. | Starting material No. | Yield (%) | Optical purity (%ee) |
|---|---|---|---|
| 20-8 | (33) | 17 | 16 |
| 20-9 | (29) | 21 | 44 |
| 20-10 | (38) | 100 | 26 |
| 20-11 | (23) | 82 | 93 |
| 20-12 | (40) | 100 | 93 |
| 20-13 | (39) | 39 | 35 |
| 20-14 | (41) | 92 | 93 |
| 20-15 | (37) | 100 | 74 |

In the above table, reaction conversion rates are listed in the columns of Yield of Examples 20-10 to 20-15.

The reaction conversion rate was calculated based on [LC area value of compound (8)]/[Sum of LC area values of compounds (8) and (3)]×100%.

The optical purities of the reaction products in Example 20-1, 20-3, 20-6, 20-7, and 20-9 were obtained by concentrating 5 mL of the acetonitrile diluted solution used for the quantitative analysis, analyzing the obtained residue in accordance with the analysis conditions described in Example 2, and calculating.

The optical purities of the reaction products in Example 20-2, 20-4, and 20-5 were obtained by deriving each reaction product into the compound (1) in accordance with the methods described below and thereafter calculating the optical purities in accordance with the analytical conditions described in Example 2.

Example 20-2: 5 mL of the acetonitrile diluted solution used for the quantitative analysis was concentrated, and 2.0 g of methanol, 0.1 g of potassium carbonate, and 0.5 g of water were added to the obtained residue, followed by stirring the resultant mixture at room temperature for 0.5 hour. After completion of the stirring, the reaction liquid was concentrated under reduced pressure and the obtained residue was used for the analysis of the optical purity.

Example 20-4: 5 mL of the acetonitrile diluted solution used for the quantitative analysis was concentrated, and 2.0 g of ethanol and 0.10 g of hydrazine monohydrate were added to the obtained residue, followed by stirring the resultant mixture at 80°C for 80 minutes. After completion of the stirring, the reaction liquid was concentrated under reduced pressure and the resultant residue was used for the analysis of the optical purity.

Example 20-5: 5 mL of the acetonitrile diluted solution used for the quantitative analysis was concentrated, and 0.5 g of N,N-dimethylformamide and 0.25 g of morpholine were added to the obtained residue, followed by stirring the resultant mixture at room temperature for 70 minutes. After completion of the stirring, the reaction liquid was concentrated under reduced pressure and the resultant residue was used for the analysis of the optical purity.

The optical purities of the reaction product in Example 20-8 was obtained by deriving the reaction product into the compound [(R)-25] in accordance with the methods described below and thereafter calculating the optical purities in accordance with the analytical conditions of LC-2.

Example 20-8: 5 mL of the acetonitrile diluted solution used for the quantitative analysis was concentrated, and 3.0 g of ethanol and 0.1 g of hydrazine monohydrate were added to the obtained residue, followed by stirring the resultant mixture at 80°C for 1 hour. After completion of the stirring, the reaction liquid was concentrated under reduced pressure and the obtained residue was used for the analysis of the optical purity.

The optical purity of the reaction products in Example 20-10 was obtained by concentrating 5 mL of the acetonitrile diluted solution used for the quantitative analysis, and calculating the optical purities of the obtained residue in accordance with the analytical conditions of LC-4.

The optical purities of the reaction products in Examples 20-11, 20-12, 20-13, and 20-14 were obtained by deriving each reaction product into the compound [(R)-21] in accordance with the methods described below and thereafter calculating the optical purities in accordance with the analytical conditions of LC-3.

Example 20-11: 10 mL of the acetonitrile diluted solution used for the quantitative analysis was concentrated, and 0.5 g of tetrahydrofuran and 0.1 g (1 mol/l) of tetrahydrofuran solution of tetra-normal-butyl ammonium fluoride were added to the obtained residue, followed by stirring the resultant mixture at room temperature for 2 hours. After completion of the stirring, the reaction liquid was concentrated under reduced pressure and the obtained residue was used for the analysis of the optical purity.

Example 20-12: 5 mL of the acetonitrile diluted solution used for the quantitative analysis was concentrated, and 0.5 g of ethanol and 10 mg of 10% by mass palladium carbon were added to the obtained residue, followed by stirring the reaction mixture under hydrogen atmosphere at room temperature for 6 hours. After completion of the stirring,

the palladium carbon was separated by filtration and the obtained filtrate was concentrated under reduced pressure, followed by using the obtained residue for the analysis of the optical purity.

Example 20-13: 5 mL of the acetonitrile diluted solution used for the quantitative analysis was concentrated, and 0.5 g of methanol and 0.1 g of 1% by mass sodium hydroxide aqueous solution were added to the obtained residue, followed by stirring the resultant mixture at room temperature for 2 hours. After completion of the stirring, the reaction liquid was concentrated under reduced pressure and the obtained residue was used for the analysis of the optical purity.

Example 20-14: 5 mL of the acetonitrile diluted solution used for the quantitative analysis was concentrated, and 0.5 g of methanol and 50 mg of hydrochloric acid (1 M) were added to the obtained residue, followed by stirring the resultant mixture at room temperature for 2 hours. After completion of the stirring, the reaction liquid was concentrated under reduced pressure and the obtained residue was used for the analysis of the optical purity.

The optical purities of the reaction products in Example 20-15 were obtained by deriving each reaction product into the compound [(R)-35] in accordance with the methods described below and thereafter calculating the optical purities in accordance with the analytical conditions of LC-4.

Example 20-15: 5 mL of the acetonitrile diluted solution used for the quantitative analysis was concentrated, and 1.0 g of tetrahydrofuran and 50 mg (1 mol/l) of tetrahydrofuran solution of tetra-normal-butyl ammonium fluoride were added to the obtained residue, followed by stirring the resultant mixture at room temperature for 7 hours. After completion of the stirring, the reaction liquid was concentrated under reduced pressure and the obtained residue was used for the analysis of the optical purity.

## INDUSTRIAL APPLICABILITY

The present invention is useful for the method for industrially producing the optically active alcohol compounds as the intermediates of the medical products.

## Claims

**1.** A method for producing an optically active alcohol compound of Formula (8):

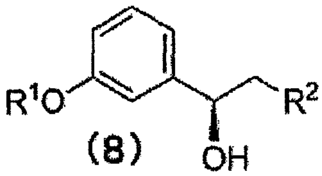

(8)

[in the formula, $R^1$ is a hydrogen atom, $C_{1-6}$ alkyl, $(C_{1-6})$ alkyl optionally substituted with $R^3$, $-C(O)R^8$, or $-Si(R^{12a})(R^{12b})R^{12}$; and
$R^2$ is cyano or $-CH_2N(R^5)R^4$;
$R^3$ is $C_{1-6}$ alkoxy, phenyl, or $C_{3-8}$ cycloalkyl;
$R^4$ is a hydrogen atom, $-C(O)R^6$, or $-C(O)OR^7$;
$R^5$ is a hydrogen atom or $C_{1-6}$ alkyl, or $R^5$ optionally forms a 5- to 7-membered ring together with a nitrogen atom to which $R^4$ and $R^5$ are bonded by forming a $C_{4-6}$ alkylene chain together with $R^4$, and in this case the alkylene chain is optionally substituted with one or more selected from the group consisting of $C_{1-6}$ alkyl, $(C_{1-6})$ alkyl optionally substituted with Y, phenyl, and an oxo group, or the alkylene chain optionally forms phenyl together with carbon atoms to which two substituents each bond when the two substituents exist at adjacent positions on the alkylene chain;
$R^6$ is $C_{1-6}$ alkyl, $(C_{1-6})$ alkyl optionally substituted with a halogen atom, or phenyl;
$R^7$ is $C_{1-6}$ alkyl or $-CH_2R^{13}$;
$R^8$ is a hydrogen atom, $C_{1-6}$ alkyl, $(C_{1-6})$ alkyl optionally substituted with a halogen atom, phenyl, or phenyl substituted with (Z)p;
$R^{12}$, $R^{12a}$, and $R^{12b}$ each are independently $C_{1-6}$ alkyl or phenyl;
$R^{13}$ is phenyl or 9-fluorenyl;
Y is a halogen atom;
Z is a halogen atom or $C_{1-6}$ alkoxy; and
p is an integer of 1, 2, 3, 4, or 5];

the method **characterized by** comprising the step of:

reacting a compound of Formula (3):

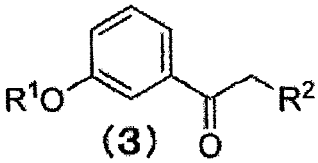

(3)

(in the formula, $R^1$ and $R^2$ are the same meaning as Formula (8)); with a reducing agent in the presence of an optically active ruthenium catalyst selected from the group consisting of a compound of Formula (4):

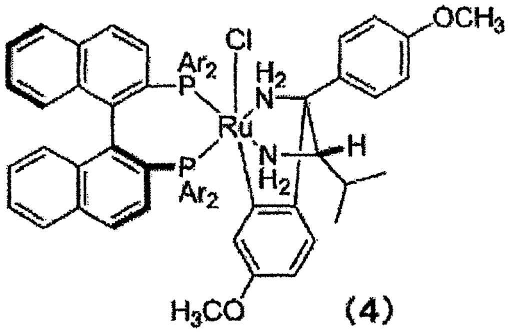

(4)

(in the formula, Ar is 3,5-dimethylphenyl), a compound of Formula (5):

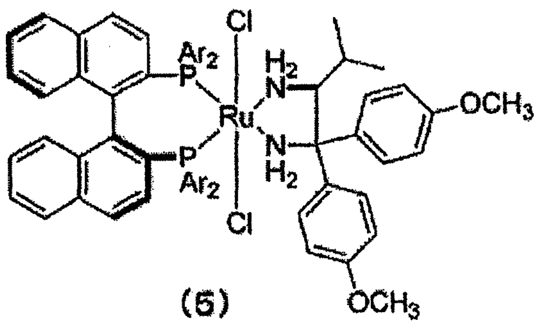

(5)

(in the formula, Ar is 3,5-dimethylphenyl), and a compound of Formula (6):

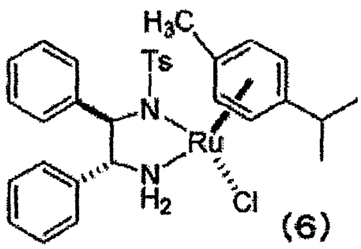

(6)

(in the formula, Ts is paratoluenesulfonyl) or an optically active oxazaborolidine compound of Formula (7),

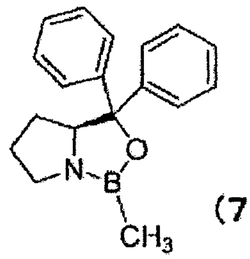

(7)

2. The method for producing the optically active alcohol compound according to claim 1, wherein $R^1$ is a hydrogen atom, $(C_{1-6})$ alkyl optionally substituted with $R^3$, $-C(O)R^8-$, or $-Si(R^{12a})(R^{12b})R^{12}$.

3. The method for producing the optically active alcohol compound according to claim 2, wherein the reaction is carried out in the presence of the optically active ruthenium catalyst of Formula (4).

4. The method for producing the optically active alcohol compound according to claim 3, wherein
$R^2$ is $-CH_2N(R^5)R^4$;
$R^4$ is $-C(O)R^6$ or $-C(O)OR^7$;
$R^6$ is $C_{1-6}$ alkyl or $(C_{1-6})$ alkyl optionally substituted with a halogen atom; and
$R^8$ is a hydrogen atom, $C_{1-6}$ alkyl, or $(C_{1-6})$ alkyl optionally substituted with a halogen atom.

5. The method for producing the optically active alcohol compound according to claim 4, wherein
$R^1$ is $(C_{1-6})$ alkyl optionally substituted with $R^3$, or $-Si(R^{12a})(R^{12b})R^{12}$;
$R^3$ is phenyl, or $C_{3-8}$ cycloalkyl;
$R^5$ is a hydrogen atom, or $R^5$ optionally forms a 5-membered ring together with a nitrogen atom to which $R^4$ and $R^5$ are bonded by forming a $C_4$ alkylene chain together with $R^4$, and in this case the alkylene chain is optionally substituted with an oxo group, or the alkylene chain optionally forms phenyl together with carbon atoms to which two substituents each bond when the two substituents exist at adjacent positions on the alkylene chain;
$R^6$ is $(C_{1-6})$ alkyl optionally substituted with a halogen atom; and
$R^{13}$ is 9-fluorenyl.

6. The method for producing the optically active alcohol compound according to claim 5, wherein
$R^1$ is $(C_{1-6})$ alkyl optionally substituted with $R^3$;
$R^3$ is $C_{3-8}$ cycloalkyl;
$R^4$ is a $-C(O)OR^7$;
$R^5$ is a hydrogen atom; and
$R^7$ is $C_{1-6}$ alkyl.

7. The method for producing the optically active alcohol compound according to any one of claims 3 to 6, wherein the reducing agent is hydrogen gas.

8. The method for producing the optically active alcohol compound according to claim 2, wherein the reaction is carried out in the presence of the optically active ruthenium catalyst of Formula (6).

9. The method for producing the optically active alcohol compound according to claim 8, wherein $R^6$ is $C_{1-6}$ alkyl or $(C_{1-6})$ alkyl optionally substituted with a halogen atom.

10. The method for producing the optically active alcohol compound according to claim 9, wherein
$R^1$ is a hydrogen atom, $(C_{1-6})$alkyl optionally substituted with $R^3$, $-C(O)R^8$, or $-Si(R^{12a})(R^{12b})R^{12}$;
$R^5$ is a hydrogen atom, or $R^5$ optionally forms a 5-membered ring together with a nitrogen atom to which $R^4$ and $R^5$ are bonded by forming a $C_4$ alkylene chain together with $R^4$, and in this case the alkylene chain is optionally substituted with an oxo group, or the alkylene chain optionally forms phenyl together with carbon atoms to which two substituents each bond when the two substituents exist at adjacent positions on the alkylene chain;
$R^6$ is $(C_{1-6})$ alkyl optionally substituted with a halogen atom;
$R^7$ is $C_{1-6}$ alkyl;
$R^8$ is phenyl; and
$R^{13}$ is 9-fluorenyl.

**11.** The method for producing the optically active alcohol compound according to claim 10, wherein
$R^1$ is $C_{1-6}$ alkyl optionally substituted with $R^3$;
$R^2$ is cyano; and
$R^3$ is $C_{3-8}$ cycloalkyl.

**12.** The method for producing the optically active alcohol compound according to any one of claims 8 to 11, wherein the reducing agent is formic acid.

**13.** A compound of Formula (3'):

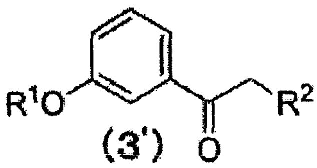

(3')

(in the formula, $R^1$ is $C_1$ alkyl substituted with $R^3$;
$R^2$ is cyano; and
$R^3$ is cyclohexyl).

**14.** A method for producing a compound of Formula (3"):

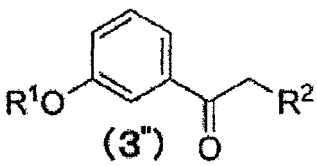

(3")

(in the formula, $R^1$ is a cyclohexylmethyl group; and
$R^2$ is $-CH_2NHC(O)O(C_4H_9\text{-t})$ or $-CH_2NHCOCF_3$);

the method **characterized by** comprising the step of:

reacting an alcohol compound of Formula (44):

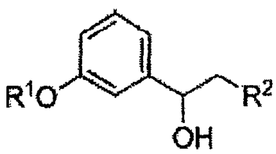

(44)

(in the formula, $R^1$ and $R^2$ are the same meaning as Formula (3")) with an oxidizing agent in the presence of N-hydroxy-2-azaadamantane of Formula (43):

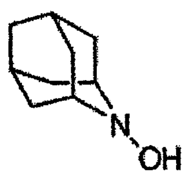

(43)

## INTERNATIONAL SEARCH REPORT

International application No.
PCT/JP2014/065771

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07C213/00, B01J31/02, B01J31/22, C07B53/00, C07C217/64, C07C231/18, C07C233/18, C07C253/30, C07C255/37, C07C269/06, C07C271/16, C07D209/48, C07F7/18, C07B61/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y<br>X | JP 2011-512321 A (Acucela Inc.), 21 April 2011 (21.04.2011), claims; paragraphs [0395] to [0403], [0513] to [0531], [1200] to [1232], [1284] to [1289] & US 2009/0326074 A1 & EP 2091955 A1 & WO 2009/045479 A1 | 1-12,14<br>13 |
| Y<br>A | JP 2010-37332 A (Erick M. Carreira), 18 February 2010 (18.02.2010), paragraphs [0192] to [0195] & US 2011/0137049 A1 & EP 2311846 A1 & WO 2010/004957 A1 | 1-3,5-10,12<br>4,11,13,14 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 September, 2014 (04.09.14) | 16 September, 2014 (16.09.14) |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2014/065771

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | MATSUMURA,K. et al., Chiral Ruthenabicyclic Complexes: Precatalysts for Rapid, Enantioselective, and Wide-Scope Hydrogenation of Ketones, Journal of the American Chemical Society, 2011, Vol.133, p.10696-10699 | 1-7<br>8-14 |
| Y<br>A | OHKUMA,T. et al., Asymmetric Hydrogenation of Alkenyl, Cyclopropyl, and Aryl Ketones. $RuCl_2$ (xylbinap)(1,2-diamine) as a Precatalyst Exhibiting a Wide Scope, Journal of the American Chemical Society, 1998, Vol.120, p. 13529-13530 | 1,2<br>3-14 |
| Y<br>A | WANG,F. et al., Asymmetric Transfer Hydrogenation of Ketones Catalyzed by Hydrophobic Metal-Amido Complexes in Aqueous Micelles and Vesicles, The Journal of Organic Chemistry, 2005, Vol.70, p.9424-9429 | 1,2,8-12<br>3-7,13,14 |
| Y<br>A | JP 2012-513966 A (Novartis AG.), 21 June 2012 (21.06.2012), claims; paragraphs [0140] to [0145] & US 2010/0168229 A1 & EP 2382172 A1 & WO 2010/072798 A1 | 1,2,8-12<br>3-7,13,14 |
| Y<br>A | DEGNI,S. et al., Highly catalytic enantioselective reduction of aromatic ketones using chiral polymer-supported Corey, Bakshi, and Shibata catalysts, Tetrahedron: Asymmetry, 2004, Vol.15, p.1495-1499 | 1,2<br>3-14 |
| Y<br>A | JP 2011-153076 A (Nissan Chemical Industries, Ltd.), 11 August 2011 (11.08.2011), claims; paragraphs [0070] to [0081] & WO 2009/145323 A1 | 14<br>1-13 |

Form PCT/ISA/210 (continuation of second sheet) (July 2009)

INTERNATIONAL SEARCH REPORT

International application No.
PCT/JP2014/065771

Continuation of A. CLASSIFICATION OF SUBJECT MATTER
(International Patent Classification (IPC))

*C07C213/00*(2006.01)i, *B01J31/02*(2006.01)i, *B01J31/22*(2006.01)i, *C07B53/00*(2006.01)i, *C07C217/64*(2006.01)i, *C07C231/18*(2006.01)i, *C07C233/18*(2006.01)i, *C07C253/30*(2006.01)i, *C07C255/37*(2006.01)i, *C07C269/06*(2006.01)i, *C07C271/16*(2006.01)i, *C07D209/48*(2006.01)i, *C07F7/18*(2006.01)i, *C07B61/00*(2006.01)n

(According to International Patent Classification (IPC) or to both national classification and IPC)

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009045479 A